# EUROPEAN PATENT SPECIFICATION

(11) **EP 4 021 383 B1**
(45) Date of publication and mention of the grant of the patent: **29.04.2026**
(21) Application number: 20764643.1
(22) Date of filing: 28.08.2020
(51) Int. Cl.: A61K 8/11, A61F 2/02, A61K 8/73, A61K 8/81, A61K 8/88, A61K 9/14, A61K 47/69, A61Q 17/04, A61Q 19/08, B82Y 30/00, A61K 9/51, A61K 8/02

(54) **BIOADHESIVE PARTICLES COMPRISING ACTIVE AGENTS, AND USES THEREOF**
BIOADHÄSIVE PARTIKEL MIT WIRKSTOFFEN UND DEREN VERWENDUNGEN
PARTICULES BIOADHÉSIVES COMPRENANT DES AGENTS ACTIFS ET LEURS UTILISATIONS

(30) Priority: 29.08.2019 EP 19306047
(43) Date of publication of application: 06.07.2022
(73) Proprietor: SKINOSIVE, 75013 Paris (FR)
(72) Inventor: POULETTY, Philippe, 75005 PARIS (FR); RAULT, Isabelle, 38300 BOURGOIN-JALLIEU (FR); CARNIATO, Denis, 91460 MARCOUSSIS (FR)
(74) Representative: Cabinet Becker et Associés
(86) International application number: PCT/EP2020/074148
(87) International publication number: WO 2021/038084

(56) References cited:
- US-A1- 2016 136 105
- US-A1- 2017 112 949
- US-A1- 2018 214 564
- US-A1- 2018 256 480
- BENJAMIN HOREV ET AL: "pH-Activated Nanoparticles for Controlled Topical Delivery of Farnesol To Disrupt Oral Biofilm Virulence", ACS NANO, vol. 9, no. 3, 24 March 2015 (2015-03-24), US, pages 2390 - 2404, XP055414641, ISSN: 1936-0851, DOI: 10.1021/nn507170s

## Description

### TECHNICAL FIELD

The present invention relates to a set of particles comprising active agent-loaded bioadhesive particles. It also relates to a composition comprising the same and, more particularly, to a cosmetic or a sunscreen composition. It also relates to the use of the set of particles in medicine, in particular for treating or preventing skin and mucosal diseases or conditions.

### TECHNICAL BACKGROUND

The development of nano- and micro-particles has been a burgeoning field over the last two decades, and the role of such materials in the society of the twenty-first century is being more and more prominent. The main interest of these small objects lies in the properties stemming from their morphology and size. More particularly, the unusual interactions of these particles with the living make them powerful tools for diagnostic or prophylactic applications, or in drug delivery. Also, the widespread use of such materials by cosmetic manufacturers is the direct result of their atypical physical properties such as color, transparency, or solubility. Beyond their intrinsic properties, their use as carriers, through encapsulation or association with active agents, allows to combine effects or reveal unexpected ones, and accordingly, to broaden applications.

Nano- and micro-particles also appear as a relevant tool to address the shortcomings in the field of UV-protection. UVB radiation is considered to be the major factor responsible for most harmful effects of solar exposure, resulting in serious skin damages from an accelerated skin ageing to a skin cancer. The negative impact of UVA has also been deeply investigated. As a result, the development of new sunscreens providing effective protection throughout the whole UV radiation spectrum has become a prime concern and a major issue. Many sunscreens are nowadays available on the market. Despite an undeniable efficacy, it has been demonstrated that active agents of these sunscreens can penetrate through the skin into epidermal cells, and have also been detected in urine or breast milk. Small and non-adhesive nanoparticles have been developed for sunscreen compositions and are currently available on the market. However, such materials have also proven to be skin-penetrating. In this regard, bioadhesive particles have been designed to adhere to the skin and to encapsulate active agents, such that neither the particles nor the active agents penetrate the skin. However, such particles display some weaknesses, particularly a moderate bioadhesion and a complex manufacturing process. 2018/0256480 A discloses nanoparticles containing an hydrophobic core and coated with hyperbranched polymers. The core may include one or more active agents.

Therefore, there remains a need to produce efficient active agent-loaded bioadhesive particles, which are easy to produce.

### SUMMARY OF THE INVENTION

In this respect, the inventors have developed bioadhesive particles comprising a polymer core and functional group(s), both being covalently linked to each other, directly or through a linear linker. The bioadhesive particles additionally comprise one or more active agents, which can be encapsulated within the particle core and/or associated to the surface of the particle. The bioadhesive particles can interact with a tissue, a cell, a cell surface, an intracellular or an extracellular material, through their functional group(s), which make them particularly suitable as carrier in many applications, from therapeutic to diagnostic or prophylactic applications, but also in cosmetics, and more specifically in sunscreen compositions, make-up and tattoo applications.

Thus, the present invention relates to a set of particles comprising at least one bioadhesive particle represented by the following formula (I):

{A-[(B)n-C]m} (I),

in which:
- A represents a polymer core,
- B represents a linear linker,
- C represents a functional group chosen from an aldehyde, an acetal, thiocetal, a thiol, a maleimide, a Mickael acceptor, a vinylsulfone, a sulfonylaziridine, an epoxide, a haloacetyl, an isocyanate, an isothiocyanate, a N-hydroxysuccinimide ester, a N-hydroxysulfosuccinimide ester, a hydroxy, an amino, an ammonium, a guanidinium, a carboxylic acid, a carboxylic ester, an anhydride, a sulfonic acid, folic acid, biotin, streptavidin, avidin, antibodies, single chain antibodies or fragments thereof,
- n is 1, and
- m is an integer from 1 to 10²⁵, preferably from 1 to 2000, and
where said bioadhesive particle encapsulates or is attached to at least one active agent X, which is a UV filter.

It also relates to a composition comprising a set of particles as defined herein, and at least one acceptable excipient.

It further relates to a cosmetic use of a composition as defined herein, for combatting and/or reducing the signs of cutaneous ageing, such as the formation of wrinkles and/or fine lines, skin sagging, loss of firmness, loss of radiance and/or evenness of the complexion, and/or for reinforcing the skin barrier.

A further object of the present invention is a cosmetic process for combatting and/or reducing the signs of cutaneous ageing, such as the formation of wrinkles and/or fine lines, skin sagging, loss of skin firmness, loss of radiance and/or evenness of the complexion, and/or for reinforcing the skin barrier, comprising applying topically to the skin, a composition as defined herein.

Another object of the present invention is a kit comprising:
- a composition as defined herein,
- a washing composition, preferably a powder, a shampoo, a soap, a lotion, a solution, a solid, a scrubbing, a scraper, a mousse, a foam, a syndet, a gel, a shower gel, a spray, a mist, a wax, a strip, an enzyme composition, a detergent composition, or a woven or non-woven fabric, and,
- optionally an instruction guide.

### DETAILED DESCRIPTION OF THE INVENTION

### Definitions

According to the present invention, the terms below have the following meanings:
The term "homopolymer" generally refers to a polymer that is composed of identical monomers.

The term "copolymer" generally refers to a polymer that is composed of two or more different monomers. The copolymer can be of any form, such as random, block, or graft. The copolymers can have any end-group.

The term "residue of polymer" refers to a polymer deprived of one or more of its functional groups (such as -NH₂, -COOH, -OH), or atom(s) thereof (such as H or -OH).

The term "mean diameter" of particles generally refers to the statistical mean particle size (diameter) of the particles in a population of particles. As used herein, the diameter of a non-spherical particle may refer to the largest linear distance between two points on the surface of the particle. Mean particle size can be measured using methods known in the art, such as Dynamic Light Scattering, Scanning Electron Microscopy, or Transmission Electron Microscopy.

The term "monodisperse" (or "homogeneous size distribution"), relative to a population of particles, describes a population of particles where all of the particles are the same or nearly the same size. As used herein, a monodisperse distribution refers to a particle distribution in which 90% or more of the distribution lies within 15% of the median particle size, more preferably within 10% of the median particle size, most preferably within 5% of the median particle size. The terms "uniform size" or "homogenous size" may qualitatively be used to describe a monodisperse population of particles.

On the contrary, the term "polydisperse", relative to a population of particles, describes a population of particles where the size distribution of said population varies in a large degree. Particularly, a polydisperse distribution refers to a particle distribution that is not comprised in the ranges of distribution as above defined for a monodisperse distribution. The terms "nonuniform size" or "heterogenous size" may qualitatively be used to describe a polydisperse population of particles.

The term "biodegradable material" refers to a material that will degrade or erode under physical, chemical, and/or biological conditions to smaller units or chemical species that are capable of being metabolized, eliminated, or excreted by the subject. The degradation time and speed are a function of composition and/or morphology. Degradation time can be from hours to weeks.

The terms "biocompatible material" and "biologically compatible material" refer to a material that is, along with any metabolites or degradation products thereof, generally non-toxic to the recipient, and do not cause any significant adverse effects to the recipient. Generally speaking, biocompatible materials are materials which do not elicit a significant inflammatory or immune response when administered to a patient.

### Particles

The present invention relates to bioadhesive particles comprising a polymer core functionalized with at least one functional group, which is linked to the polymer core directly or through a linear linker. The linear linker may be polymeric or not. More particularly, the polymer core comprises at least one polymer residue (i.e., at least one polymer deprived of one or more of its functional groups) with at least one functional group linked to the polymer core directly or through a linear linker able to render the particle bioadhesive.

The bioadhesive particle of the invention can typically be represented by the following formula (I):

{A-[(B)ₙ-C]ₘ} (I),

in which:
- A represents a polymer core,
- B represents a linear linker,
- C represents a functional group chosen from an aldehyde, an acetal, thiocetal, a thiol, a maleimide, a Mickael acceptor, a vinylsulfone, a sulfonylaziridine, an epoxide, a haloacetyl, an isocyanate, an isothiocyanate, a N-hydroxysuccinimide ester, a N-hydroxysulfosuccinimide ester, a hydroxy, an amino, an ammonium, a guanidinium, a carboxylic acid, a carboxylic ester, an anhydride, a sulfonic acid, folic acid, biotin, streptavidin, avidin, antibodies, single chain antibodies or fragments thereof,
- n is 1, and
- m is an integer from 1 to 10²⁵, preferably from 1 to 2000, and
where said bioadhesive particle encapsulates or is attached to at least one active agent X, which is a UV filter.

According to the invention, A represents a polymer core. A polymer core comprises at least one polymer representing the core of the particle. Particularly, the polymer core A comprises at least one residue of polymer as above defined.

The polymer may be a homopolymer or a copolymer. The polymer may be natural, semi-synthetic, hemi-synthetic, or synthetic.

Examples of semi-synthetic, hemi-synthetic or synthetic polymers include, but are not limited to, poly(hydroxy acids) such as poly(lactic acid), poly(glycolic acid), and poly(lactic acid-co-glycolic acid), poly(lactide), poly(glycolide), poly(lactide-co-glycolide), polyanhydrides, polyorthoesters, polyamides, polycarbonates, polyalkylenes such as polyethylene and polypropylene, polyalkylene glycols such as poly(ethylene glycol), polyalkylene oxides such as poly(ethylene oxide), poly(lactic acid)-b-poly(ethyleneglycol), polyalkylene terepthalates such as poly(ethylene terephthalate), polyvinyl alcohols, polyvinyl ethers, polyvinyl esters, polyvinyl halides such as poly(vinyl chloride), polyvinylpyrrolidone, polysiloxanes, poly(vinyl alcohols), poly(vinyl acetate), polystyrene, polyurethanes and co-polymers thereof, derivatized celluloses such as alkyl cellulose, hydroxyalkyl celluloses, cellulose ethers, cellulose esters, nitro celluloses, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, hydroxypropyl methylcellulose acetate phthalate, carboxylethyl cellulose, cellulose triacetate, and cellulose sulfate sodium salt (jointly referred to herein as "synthetic celluloses"), polymers of acrylic acid, methacrylic acid or copolymers or derivatives thereof including esters, copolymers of acrylates and ammonium methacrylate, poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate) (jointly referred to herein as "polyacrylic acids"), poly(butyric acid), poly(valeric acid), and poly(lactide-co-caprolactone), copolymers and blends thereof.

Examples of natural polymers include, but are not limited to, proteins such as albumin, collagen, gelatin and prolamines (e.g. zein), and polysaccharides such as alginate, dextran, chitosan, cellulose derivatives and polyhydroxyalkanoates (e.g. polyhydroxybutyrate) and microbial anatoxins.

In a particular embodiment, said polymer is biodegradable.

In a particular embodiment, A comprises at least one residue of a polymer chosen from poly(lactic acid), poly(glycolic acid), poly(lactic co glycolic), polyacrylic acid, acrylic acid copolymers, poly(methacrylic acid), polyalkylacrylates (e.g. polymethacrylates, polybutyl acrylates), polyalkylcyanoacrylates, polyvinylacetate phtalate, cellulose, ethylcellulose, cellulose acetate, cellulose acetate phthalate, hydroxyethylcellulose, carboxymethylcellulose, polyvinyl alcohol, polycaprolactone, polycaprolactone crosspolymers, styrene copolymers, nylons, polymethylsilsesquioxane, poly(2-ethyl-2-oxazoline), povidone, alginate, chitosan, dextran, poly(methylvinylether/maleic anhydride) copolymers, poly(lactic acid)-b-poly(ethyleneglycol), and combinations thereof.

In a preferred embodiment, A comprises at least one residue of a polymer chosen from poly(lactic acid) and poly(lactic co glycolic). As used herein poly(lactic acid) may be represented by "PLA" and poly(lactic co glycolic) may be represented by "PLGA".

The lactic acid units in poly(lactic acid), poly(lactic co glycolic), or poly(lactic acid)-b-poly(ethyleneglycol) may be D-lactic acid units, L-lactic acid units, or a mixture thereof. In particular, the poly(lactic acid) may be poly(D,L-lactic acid), poly(L-lactic acid) or poly(D-lactic acid).

According to the invention, m represents the number of [(B)ₙ-C] moieties. m may also represent the number of functional groups C. In a particular embodiment, m is comprised between 1 and 2000, preferably between 100 and 1000, more preferably between 150 and 500, and even more preferably between 150 and 250. m may be further comprised between 10 and 10¹⁸, between 100 and 10¹⁵, between 1000 and 10¹², between 1000 and 10⁸, between 1000 and 10⁵, or between 100 and 10³.

In the formula (I), C represents a functional group as defined above. As used herein, a "functional group" is a group that is able to react with or bound to tissues, cells, intracellular or extracellular materials, making the particle of formula (I) bioadhesive.

In a particular embodiment of the invention, C is chosen from an aldehyde, an acetal, thiocetal, a thiol, a maleimide, a Mickael acceptor, a vinylsulfone, a sulfonylaziridine, an epoxide, a haloacetyl, an isocyanate, an isothiocyanate, a N-hydroxysuccinimide ester, a N-hydroxysulfosuccinimide ester, a hydroxy, an amino, an ammonium, a guanidinium, a carboxylic acid, a carboxylic ester, an anhydride, a sulfonic acid, folic acid, biotin, streptavidin, avidin, antibodies, single chain antibodies or fragments thereof. In another embodiment, C is a cell adhesion peptide, for instance a RGD peptide (arginylglycylaspartic acid), or a HAV peptide. In a further particular embodiment, C further includes any derivatives of such functional groups as above detailed.

For instance, the term "maleimide" (Mal) refers to the maleimide group and any derivative thereof. In particular, the maleimide (Mal) includes the two following groups of formula (Mal 1), and (Mal 2):

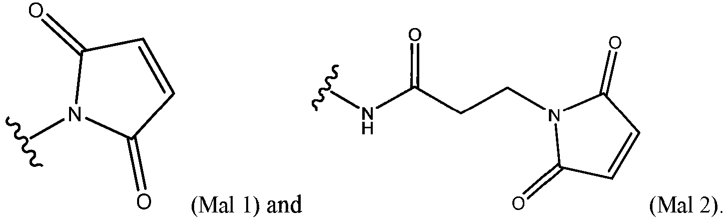

In a preferred embodiment of the invention, C is a maleimide (also named "Mal") or a derivative of maleimide as above disclosed.

In a further preferred embodiment of the invention C is a vinyl sulfone (also named "VS").

In a further particular embodiment, C represents a native functional group.

As used herein, a "native functional group" of a polymer is a functional group which is intrinsically present in the structure of the polymer and thus has not been converted into another functional group. For instance, hydroxy groups are native functional groups of cellulose, and carboxylic acids (or carboxylate) are native functional groups of a polymethacrylic acid.

In a further particular embodiment, C represents a modified functional group.

As used herein, a "modified functional group" of a polymer is a functional group which results from the conversion of a native functional group as defined herein into another functional group, e.g., an aldehyde group resulting from oxidation of a hydroxy group of cellulose or a biotin linked to a hydroxy group of cellulose.

### Particles of formula (I):

In a particular embodiment, n is 1. According to this embodiment, the bioadhesive particles of formula (I) comprise a linear linker B.

In such an embodiment, bioadhesive particles of formula (I) can be represented by the following formula:

{A-[B-C]ₘ} (I),

in which A, B, C, and m are such as defined herein, and said bioadhesive particles encapsulate or are attached to at least one active agent X, which is a UV filter.

In a particular embodiment of formula (I), C is chosen from a thiol, a maleimide, a Mickael acceptor, a vinylsulfone, a sulfonylaziridine, an epoxide, a haloacetyl, an isocyanate, an isothiocyanate, a N-hydroxysuccinimide ester, a N-hydroxysulfosuccinimide ester, a hydroxy, an amino, a guanidinium, an ammonium, a carboxylic acid, a carboxylic ester, an anhydride, a sulfonic acid, folic acid, biotin, streptavidin, avidin, antibodies, single chain antibodies or fragments thereof, and derivatives thereof. Preferably, C is a maleimide or a derivative thereof, or a vinyl sulfone, more preferably a maleimide or a derivative thereof.

A bioadhesive particle of formula (I) thus comprises a moiety A-[B-C]ₘ comprising a polymer core A and m moieties [B-C] that are directly and covalently bound to the polymer core (A) through B, each [B-C] being independently a linear linker (B) bound to one or more functional groups C, identical or different, and in which at least one active agent (X) is encapsulated or attached thereto. In the formula (I), m represents the number of linear linkers B covalently bound to A and to one or more functional groups (C). The m moieties [B-C] are linked to one or more residues of polymer of the polymer core A.

According to the invention, B represents a linear linker. A "linear linker" refers to a linker which is not branched. For instance, a "linear linker" can be a linear polymer (or a residue thereof) but cannot be a branched or hyperbranched polymer. In a preferred embodiment, the linear linker is not hyperbranched polyglycerol.

The linear linker (B) may be bound to one or more functional groups (C). In a particular embodiment, a linear linker (B) is bound to a single functional group (C). In another particular embodiment, a linear linker (B) is bound to several (i.e. two or more) functional groups (C).

In an embodiment, the linear linker B in formula (I) comprises at least one residue of polymer.

In such an embodiment, the functional group(s) C is(are) linked to the at least one residue of polymer of the linear linker B. In such an embodiment, C in formula (Ib) represents one or more functional groups of at least one residue of polymer of the linear linker (B).

The following examples are provided for information only, in order to illustrate various embodiments for the moiety [B-C].
- [B-C] can be cellulose. In this embodiment, B represents a residue of cellulose, and C represents hydroxy groups (that are native groups of cellulose);
- [B-C] can also be aldehyde-modified cellulose. In this embodiment, B represents a residue of cellulose, and C represents aldehyde groups (that are modified groups of cellulose);
- [B-C] can also be biotin-modified cellulose. In this embodiment, B represents a residue of cellulose, and C represents biotin groups (that are modified groups of cellulose);
- [B-C] can also be a PEG-Mal moiety. In this embodiment, B represents a residue of polyethylene glycol (PEG), and C represents a maleimide or a derivative thereof. Such moieties may be represented by following formulae: 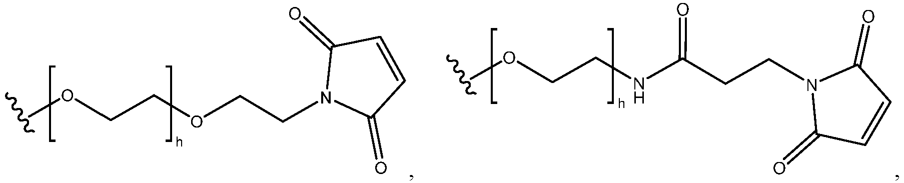 h corresponding to the number of the PEG units;
- [B-C] can also be a PEG-VS moiety. In this embodiment, B represents a residue of PEG, and C represents a VS.

In another particular embodiment, the linear linker (B) comprises at least one residue of polymer chosen from polyalkylene glycols such as poly(ethylene glycol), poly(methacrylic acid), polymethacrylates, polyalkylcyanoacrylates, polyvinylacetate phtalate, polyvinyl alcohol, povidone, and poly(methylvinylether/maleic anhydride) copolymers, and combination thereof.

In a preferred embodiment, the linear linker (B) comprises at least one residue of poly(ethylene glycol).

The poly(ethylene glycol) may for instance have a molecular weight comprised between 44 and 20 000 Da, preferably between 1 000 and 15 000 Da, more preferably between 5 000 and 10 000 Da.

In a further particular embodiment, B is a group of formula (II):

-[Y-(CH₂)_{q}]ₖ-(O-CH₂-CH₂)ₚ- (II),

in which:
- Y is selected from -O-, -NH-, and -C(O)-, preferably -C(O)-,
- p is an integer from 0 to 25, preferably from 0 to 12,
- k is 0 or 1,
- q is an integer from 1 to 10, and
- p+k is different from 0.

In a preferred embodiment, B is a group of formula (II), in which k is 0 and p is an integer from 1 to 12, preferably from 1 to 6.

In a further preferred embodiment, B is a group of formula (II) in which k is 0 and p is 1 or Y is O, q is 2, k is 1, and p is 0. According to this preferred embodiment, B is a group of formula -O-CH₂-CH₂-, namely an oxyethylene group.

In a particular embodiment, B is a group of formula (II) as defined herein and C is a maleimide or a derivative thereof or a vinylsulfone, preferably a maleimide, as above defined.

The group of formula (II) as represented above is linked to the polymer core A on its left and to a functional group C on its right. In an embodiment where n is 1 and B is a group of formula (II), a bioadhesive particle of formula (I) can be represented as follows:

{A-[[Y-(CH₂)_{q}]ₖ-(O-CH₂-CH₂)ₚ-C]ₘ},

in which A, Y, C, m, p, k, and q are as defined herein, and where said bioadhesive particle encapsulates or is attached to at least one active agent X, which is a UV filter.

In a preferred embodiment, bioadhesive particles of formula (I) as defined herein are such as:
- A comprises at least one residue of a polymer chosen from poly(lactic acid) and poly(lactic co glycolic);
- B comprises at least one residue of poly(ethylene glycol) or B is a group of formula (II) wherein k is 0 and p is 1;
- C is a maleimide or a vinyl sulfone; and
- m is comprised between 1 and 10²⁵, for instance between 10 and 10¹⁸, between 100 and 10¹⁵, between 1000 and 10¹², between 1000 and 10⁸, between 1000 and 10⁵, between 100 and 10³, between 1 and 2000, between 100 and 1000, between 150 and 500, or between 150 and 250.

Preferred examples of bioadhesive particles of formula (I) as defined herein are such as:
- A comprises at least one residue of poly(lactic acid), B comprises at least one residue of poly(ethylene glycol), C is maleimide or a derivative thereof, and m is comprised between 1 and 10²⁵, preferably between 1 and 2000 (also named "PLA-PEG-Mal");
- A comprises at least one residue of poly(lactic co glycolic), B comprises at least one residue of poly(ethylene glycol), C is vinyl sulfone, and m is comprised between 1 and 10²⁵, preferably between 1 and 2000 (also named "PLGA-PEG-VS"); and
- A comprises at least one residue of poly(lactic acid), B is a group of formula (II) wherein k is 0 and p is 1, C is a maleimide or a derivative thereof, and m is comprised between 1 and 10²⁵ , preferably between 1 and 2000 (also named "PLA-O-CH₂-CH₂-Mal").

In these more preferred examples, m may in particular be comprised between 10 and 10¹⁸, between 100 and 10¹⁵, between 1000 and 10¹², between 1000 and 10⁸, between 1000 and 10⁵, between 100 and 10³, between 1 and 2000, between 100 and 1000, between 150 and 500, or between 150 and 250.

Examples of commercial polymers suitable for preparing the bioadhesive particles of the invention may be purchased from the company Nanosoft Polymers (USA).

### Active agent X

According to the invention, a bioadhesive particle of formula (I) as defined herein encapsulates or is attached to at least one active agent X.

The terms "active agent" or "bioactive agent", as used interchangeably herein, include, without limitation, physically, physiologically or pharmacologically active substances. In the present invention, the active agent is a UV filter.

According to the invention, X is encapsulated or attached to the bioadhesive particle of formula (I). In a particular embodiment, X is encapsulated within the particles of formula (I) and/or associated with the surface of said particles. In an embodiment, X is encapsulated within the particles of formula (I). In another embodiment, X is associated with the surface of particles of formula (I).

In a particular embodiment, a bioadhesive particle may be represented by the following formula (III):

{A-[(B)ₙ-C]ₘ}.X (III),

in which:
- A represents a polymer core,
- B represents a linear linker,
- C represents a functional group,
- X represents at least one active agent,
- n is 1, and
- m is an integer from 1 to 10²⁵, preferably from 1 to 2000.

In the formula (III), the dot between "{A-[(B)ₙ-C]ₘ}" and "X" denotes an interaction between the particle and the active agent X. In a preferred embodiment, X is non-covalently associated with the particles of formula (I) (e.g. ionic bond, weak bond, H-bond, pi-stacking, electrostatic interactions, van der Waals interactions).

Particles of formula (I) may be designed to release the active agent X, for instance over a period of hours to weeks. Said release may be controlled by various parameters such as pH of the medium and the particle composition (i.e., the nature of A, B, and/or C). Particles of formula (I) can therefore be useful for delivery an active agent X. Alternatively, particles of formula (I) may be designed to substantially not release the active agent X.

Ultraviolet light filters (also referred to herein as "UV-filters" or "UV-blockers") include, but are not limited to, zinc oxide (ZnO), titanium dioxide (TiO2), avobenzone, bis-ethylhexyloxyphenol methoxyphenyl triazine (tinosorb S), mexoryl SX, helioplex, ethylhexyl methoxycinnamate (uvinul MC 80), octocrylene, oxybenzone, octisalate, homosalate, ethylhexyl triazone (uvinul T 150), cinoxate, aminobenzoic acid, padimate O, ensulizole, dioxybenzone, meradimate, sulisobenzone, trolamine salicylate, enzacamene, bisdisulizole disodium, uvasorb HEB, amiloxate, bemotrizinol, diethylamino hydroxybenzoyl hexyl benzoate (Uvinul A plus), disodium phenyl dibenzimidazole tetrasulfonate (Neo Heliopan AP), dometrizole trisiloxane (Mexoryl XL), meradimate, bisoctrizole (Tinosorb M), ecamsule, benzophenone-3 (Uvinul M40), benzophenone-4 (Uvinul MS 40), iscotrizinol (Uvosorb EHB), octyltriazone, homosalate, amiloxate, PEG-25 PABA (Uvinul P25), Polysilicone-15 (Parsol SLX), Tris Biphenyl Triazine (Tinosorb A2B), amino benzoic acid, trolamine salicylate, benzophenone 9 (Uvinul DS 49), benzophenone 8 (Dioxybenzone), octyl dimethyl 4-aminobenzoic acid (Padimite A), Polysilicone-15 and combinations thereof. Preferred UV filters are octocrylene, diethylamino Hydroxybenzoyl Hexyl Benzoate, bis-ethylhexyloxyphenol methoxyphenyl triazine, ethylhexyl triazone, bemotrizinol, and ecamsule. More preferred UV-filters are octocrylene and diethylamino Hydroxybenzoyl Hexyl Benzoate (Uvinul A Plus).

In a particular embodiment, the set of particles comprises particles of formula (I) wherein all the particles of formula (I) comprise the same UV-filter. Alternatively, the set of particles comprises particles of formula (I) wherein the particles of formula (I) comprise two or more different UV-filters, associated with a same particle and/or with different particles.

Alternatively, the particles of the set can comprise two or more different UV-filters, associated with a same particle and/or with different particles.

More preferably, X is octocrylene, diethylamino Hydroxybenzoyl Hexyl Benzoate, bis-ethylhexyloxyphenol methoxyphenyl triazine, ethylhexyl triazone, bemotrizinol, or ecamsule, and even more preferably, X is octocrylene or diethylamino Hydroxybenzoyl Hexyl Benzoate (Uvinul A Plus).

In a preferred embodiment, bioadhesive particles of formula (I) are such as:
- A comprises at least one residue of a polymer chosen from poly(lactic acid) and poly(lactic co glycolic);
- B comprises at least one residue of poly(ethylene glycol) or is a group of formula (II) wherein k is 0 and p is 1;
- C is a maleimide or a derivative thereof, or a vinyl sulfone;
- m is comprised between 1 and 10²⁵, for instance between 10 and 10¹⁸, between 100 and 10¹⁵, between 1000 and 10¹², between 1000 and 10⁸, between 1000 and 10⁵, between 100 and 10³, between 1 and 2000 between 100 and 1000, between 150 and 500, or between 150 and 250; and
- said bioadhesive particles encapsulate or are attached to at least on active agent X chosen from UV-filters.

More preferred examples of bioadhesive particles of formula are such as:
- A comprises at least one residue of poly(lactic acid), B comprises at least one residue of poly(ethylene glycol), C is maleimide or a derivative thereof, m is comprised between 1 and 10²⁵, preferably between 1 and 2000, and said bioadhesive particles encapsulate or are attached to at least on active agent X chosen from UV filters;
- A comprises at least one residue of poly(lactic co glycolic), B comprises at least one residue of poly(ethylene glycol), C is vinyl sulfone, m is comprised between 1 and 10²⁵, preferably between 1 and 2000, and said bioadhesive particles encapsulate or are attached to at least on active agent X chosen from UV filters;
   or
- A comprises at least one residue of poly(lactic acid), B is a group of formula (II) wherein k is 0 and p is 1, C is a maleimide or a derivative thereof, m is comprised between 1 and 10²⁵, preferably between 1 and 2000, and said bioadhesive particles encapsulate or are attached to at least on active agent X chosen from UV filters.

In these more preferred examples, X is preferably octocrylene or diethylamino Hydroxybenzoyl Hexyl Benzoate (Uvinul A Plus).

In these more preferred examples, m may in particular be comprised between 10 and 10¹⁸, between 100 and 10¹⁵, between 1000 and 10¹², between 1000 and 10⁸, between 1000 and 10⁵, between 100 and 10³, between 1 and 2000 between 100 and 1000, between 150 and 500, or between 150 and 250.

Interestingly, the bioadhesives particles in which a UV-filter is encapsulated or attached thereto exhibit a broader UV spectral range than the corresponding UV-filter as such.

### Physico-chemical characteristics of a particles of formula (I)

According to the invention, a particle of formula (I) is bioadhesive.

The expression "bioadhesive particle", also referred to herein as "sticky particle", denotes a particle that is able to adhere, through one or more functional groups (e.g. functional groups C as defined herein), to tissue, cells, intracellular or extracellular materials, such as proteins. More specifically, said particle bioadheres through one or more of its functional groups (e.g. functional groups C as defined herein), which are able to react with reactive groups or entities of tissues, cells (e.g. chondrocytes, osteoblasts, fibroblasts, blood cells, plasmocytes), intracellular or extracellular materials (e.g. proteins, glycoproteins, collagen, elastin, glycosaminoglycans, proteoglycans), such as a skin.

Reactive groups that can be found on tissues, cells, intracellular or extracellular materials include, but are not limited to, amine, ammonium, guanidinium, thiol, carboxylic acid, and carboxylate.

Said functional groups C may advantageously react selectively with a particular reactive group found on tissues, cells, intracellular or extracellular materials.

For instance:
- maleimide, thiol, Mickael acceptors, sulfonylazeridine, vinylsulfone, isocyanate, or thiocyanate can typically be selective to thiol groups;
- carboxylic acid, aldehyde, acetal, esters, NHS esters, sulfo-NHS esters, or anhydride can typically be selective to amine groups;
- carboxylate can typically be selective to ammonium;
- amine can typically be selective to carboxylic acid; and
- ammonium and guanidinium can typically be selective to carboxylate.

Reaction of a functional group C of a bioadhesive particle with a reactive group of tissues, cells, intracellular or extracellular materials, creates a bond such as amide, disulfide, thioether, thiocarbamate, imine, or ionic pair -NH₃⁺,⁻OOC-. The bound created between a functional group of a bioadhesive particle and a reactive group of tissue, cells, intracellular or extracellular materials may be covalent or ionic. Said bound is advantageously reversible. Said bound may be cleaved by use of a cleaving material selected from chemical and physical agents (e.g. protein, peptide (e.g. glutathione), amino acid, enzyme (e.g. cathepsin B), thiol (e.g. 2-mercaptoethanol, N-acetyl cysteine), dithiol (e.g. dithiothreitol), pH-modifier, acid, base, solvent, and/or woven or non-woven tissue. The skilled artisan is able to select an appropriate cleaving material depending on the nature of the bound and/or the composition.

In a particular embodiment, a bioadhesive particle of formula (I) is biocompatible.

A bioadhesive particle of formula (I) may have a mean diameter comprised between 1 nm and 1 mm, for instance between 50 nm and 500 µm, between 100 nm and 100 µm, between 500 nm and 1 µm, between 1 nm and 500 nm, between 1 µm and 500 µm, between 500 µm and 1 mm, between 100 nm and 50 µm, between 100 nm and 5µm, between 200 nm and 1µm, between 200 nm and 5 µm, or between 200 nm and 999 µm.

The mean diameter may be judiciously chosen by the skilled artisan depending on the intended application. For some therapeutic applications, the mean diameter is advantageously comprised between 1 nm and 999 µm, preferably between 100 nm and 50 µm, and more preferably between 200 nm and 50µm. For some cosmetic applications, the mean diameter is advantageously comprised between 1 nm and 999 µm, preferably between 100 nm and 50 µm and more preferably between 200 nm and 50 µm.

In a preferred embodiment, the polydispersity of a bioadhesive particle of formula (I) may be from about 0.05 to 5. The polydispersity of particles of the invention is measured by any methods known from a skilled person, such as gel permeation chromatography.

The bioadhesive particles of formula (I) may be monodisperse, polydisperse, unimodal and/or multimodal. In a particular embodiment, the bioadhesive particles of formula (I) are monodisperse.

### Set of particles

An object of the present invention is a set of particles comprising at least one bioadhesive particle of formula (I) as defined herein. A set of particles according to the invention refers to two or more particles, at least one of which is a bioadhesive particle of formula (I). More generally, a set of particles according to the invention refers to a plurality of particles, at least one of which is a bioadhesive particle of formula (I).

The set of particles of the invention comprises at least one bioadhesive particle of formula (I) as defined herein. In a particular embodiment, said set of particles comprises at least 20%, 30%, 40%, 50%, 60%, 70%, 80%, 90%, 99.9% or 100% by weight of bioadhesive particles of formula (I), relative to the total weight of the set.

The set of particles may further comprise particles that are different from particles of formula (I), such as polymer particles, and that optionally comprise at least one active agent, such as an active agent X as defined herein. These particles may be bioadhesive or non-bioadhesive. In a particular embodiment, these particles are bioadhesive and comprise at least one functional group, such as a functional group C as defined herein.

In a particular embodiment, at least 50%, at least 60%, at least 70%, at least 80%, at least 90%, at least 95%, at least 98%, or at least 99% by number, of the particles of the set (i.e. particles of formula (I) and particles different from particles of formula (I)) under consideration will be bioadhesive.

The set of particles according to the invention is advantageously biocompatible.

In an embodiment, the set of particles comprises particles of formula (I), each comprising one X. Alternatively, the set of particles comprises particles of formula (I), each comprising two or more X, each X being identical in the same particle and/or in different particles. Alternatively, the set of particles comprises particles of formula (I), each comprising two or more X, X being different in the same particle and/or in different particles. Accordingly, such set of particles comprising any combination of particles of formula (I) as well as any combination of active agents X may provide a synergy for therapeutic, prophylactic and/or cosmetic effects.

In an embodiment, the set of particles comprises particles of formula (I), each comprising at least one therapeutic agent as disclosed herein and at least one diagnostic agent as disclosed herein, preferably a magnetic agent or a magnetic molecule. Such set of particles is particularly suitable for magnetic guidance systems, for treating cancer.

### Methods for preparing particles

Methods for preparing polymeric particles are known in the art. Common microencapsulation techniques include, but are not limited to, spray drying, interfacial polymerization, hot melt encapsulation, phase separation encapsulation (spontaneous emulsion microencapsulation, solvent evaporation microencapsulation, and solvent removal microencapsulation), coacervation, low temperature microsphere formation, and phase inversion nanoencapsulation (PIN). A brief summary of these methods is presented below. In some embodiments, the particles are prepared using an emulsion-based technique. In particular embodiments, the particles are prepared using a double emulsion solvent evaporation technique.

### Phase Separation Microencapsulation

In phase separation microencapsulation techniques, a polymer solution is stirred, optionally in the presence of one or more active agents to be encapsulated. While continuing to uniformly suspend the material through stirring, a nonsolvent for the polymer is slowly added to the solution to decrease the polymer's solubility. Depending on the solubility of the polymer in the solvent and nonsolvent, the polymer either precipitates or phase separates into a polymer rich and a polymer poor phase. Under proper conditions, the polymer in the polymer rich phase will migrate to the interface with the continuous phase, encapsulating the active agent(s) in a droplet with an outer polymer shell.

### Spontaneous Emulsion Microencapsulation

Spontaneous emulsification involves solidifying emulsified liquid polymer droplets formed above by changing temperature, evaporating solvent, or adding chemical cross-linking agents. The physical and chemical properties of the encapsulant, as well as the properties of the one or more active agents optionally incorporated into the nascent particles, dictates suitable methods of encapsulation. Factors such as hydrophobicity, molecular weight, chemical stability, and thermal stability affect encapsulation.

### Solvent Evaporation Microencapsulation

Methods for forming microspheres using solvent evaporation techniques are described in E. Mathiowitz et al., J. Scanning Microscopy, 4:329 (1990); L.R. Beck et al., Fertil. Steril., 31:545 (1979); L.R. Beck et al, Am. J Obstet. Gynecol., 135(3) (1979); S. Benita et al., J. Pharm. Sci., 73:1721 (1984); and U.S. Patent No. 3,960,757 to Morishita et al. The polymer is dissolved in a volatile organic solvent, such as methylene chloride. One or more active agents to be incorporated are optionally added to the solution, and the mixture is suspended in an aqueous solution that contains a surface active agent such as poly(vinyl alcohol). The resulting emulsion is stirred until most of the organic solvent evaporated, leaving solid microparticles/nanoparticles. This method is useful for relatively stable polymers like polyesters and polystyrene.

### Phase Inversion Nanoencapsulation (PIN)

Nanoparticles can also be formed using the phase inversion nanoencapsulation (PIN) method, wherein a polymer is dissolved in a "good" solvent, fine particles of a substance to be incorporated, such as a drug, are mixed or dissolved in the polymer solution, and the mixture is poured into a strong non solvent for the polymer, to spontaneously produce, under favorable conditions, polymeric microspheres, wherein the polymer is either coated with the particles or the particles are dispersed in the polymer. See, e.g., U.S. Patent No. 6,143,211 to Mathiowitz, et al. The method can be used to produce monodisperse populations of nanoparticles and microparticles in a wide range of sizes, including, for example, about 100 nanometers to about 10 microns.

### Microfluidics

Nanoparticles can be prepared using microfluidic devices. A polymeric material is mixed with a drug or drug combinations in a water miscible organic solvent. The water miscible organic solvent can be one or more of the following: acetone, ethanol, methanol, isopropyl alcohol, acetonitrile and dimethyl sulfoxide (DMSO). The resulting mixture solution is then added to an aqueous solution to yield nanoparticle solution.

Other methods, well-known in the art, can be implemented to prepare particles comprising a polymer core, such as:
- Prilling encapsulation processes, which consists in formation of droplets by spraying of hotmelt material containing the active agent(s) and solidification in ambient / cool air or by chemical reactions;
- Microencapsulation by spray drying which consists spraying of a polymer solution in hot air;
- Encapsulation processes by emulsion, which consists in a stabilization of droplets formed by emulsion (o/w, w/o, double emulsion) by coacervation, reticulation, thermal gelation, solidification, interfacial or in-situ polymerization, solvent evaporation;
- Atomization which consists in breaking liquid polymer solution into small droplets which are dried; and
- Sonication process, which develops high shear of the polymer solution and very fine emulsions.

Methods for functionalizing a polymer are well-known in the art (Graft Copolymers, Hadjichristidis, et al., Encyclopedia of Polymer Science and Technology, September 2010*;* Copolymers: Synthesis, Self-Assembly, and Applications, Feng, et al., Polymers 2017, 9, 494*;* Hybrid Block Copolymers Constituted by Peptides and Synthetic Polymers: An Overview of Synthetic Approaches, Supramolecular Behavior and Potential Applications, M. Morell et al, Polymers 2013, 5, 188-224)*.*

In a particular embodiment, the polymer core of a particle of formula (I) may be subjected to a functionalization step comprising:
(a1) modifying at least one native functional group of the polymer core (e.g. oxidizing at least one alcohol group of the polymer core into aldehyde, or grafting biotin to at least one alcohol group of the polymer core).

In another embodiment, the polymer core may be subjected to a functionalization step comprising:
(a2-1) reacting at least one functional group of the polymer core with at least one linker B having at least one reactive group, the at least one functional group of the polymer core and the at least one reactive group of B being complementary, such that they are able to react together to create a bond, and
(a2-2) optionally modifying at least one native functional group of B (e.g. oxidizing at least one alcohol group of B into aldehyde, or grafting biotin to at least one alcohol group of B), to provide at least one functional group C linked to the linker B.

In another embodiment, the polymer core may be subjected to a functionalization step comprising:
(a3) reacting at least one functional group of the polymer core with at least one group [B-C] having at least one reactive group, the at least one functional group of the polymer core and the at least one reactive group of [B-C] being complementary, such that they are able to react together to create a bond.

For instance:
- a polymer core having at least one carboxylic function is typically reacted with at least one B or [B-C] having at least one alcohol or an amine group in the presence of a coupling reagent, and optionally an activating agent, in an organic solvent;
- a polymer core having at least one amine or alcohol function is coupled with at least one B or [B-C] having at least one carboxylic function in the presence of a coupling reagent, and optionally an activating agent, in an organic solvent.

Examples of coupling reagent include, but are not limited to, 1,1'-carbonyldiimidazole, dicyclohexylcarbodiimide, N-(3-Dimethylaminopropyl)-N'-ethylcarbodiimide, and combinations thereof.

Examples of organic solvent include, but are not limited to, methylene chloride, dimethylformamide (DMF), tetrahydrofuran (THF), dimethylsulfoxide (DMSO), acetonitrile, acetone, alcohol (e.g. ethanol, methanol, propanol), diethyl ether, toluene, ethyl acetate, hydrocarbons (e.g. hexane, pentane, cyclohexane), water, and mixtures thereof.

Examples of activating agent include, but are not limited, to N-hydroxysuccinimide, 1-hydroxybenzotriazole (HOBt), 7-aza-1-hydroxybenzotriazole (HOAt), and combinations thereof.

### Composition

Another object of the invention is a composition comprising a set of particles of the invention as defined herein, and at least one acceptable excipient.

In a particular embodiment, the composition according to the invention comprises from 0.01 wt% to 99 wt% of particles, preferably from 0.01 wt% to 90 wt% of particles, more preferably from 1 wt% to 70 wt%, even more preferably from 5 wt% to 50 wt%, relative to the total weight of the composition.

The composition according to the invention may in particular be in the form of a suspension, a cream, a spray, an aerosol, a butter, a stick, a gel, an ointment, a lotion, a solution, a solid, an emulsion, an oil, a lyophilizate, a milk, a powder, a paste, a wax, a mousse or a foam. The composition can be prepared according to processes known to the skilled artisan.

The composition of the invention may comprise a solvent or a dispersion medium comprising, for instance, water, ethanol, one or more polyols (e.g., glycerol, propylene glycol, and liquid polyethylene glycol), oils, such as vegetable oils (e.g., peanut oil, corn oil, sesame oil, etc.), and combinations thereof. The proper fluidity can be maintained, for example, by the use of a coating, such as lecithin, by the maintenance of the required particle size in the case of dispersion and/or by the use of surfactants. In many cases, it will be preferable to include isotonic agents, for example, sugars or sodium chloride.

Examples of excipients include, but are not limited to, surfactants, dispersants, emulsifiers, pH modifying agents, pH-buffers, viscosity modifying agents, preservatives, polymerizers, pigments, colorants, stabilizing agents, glidants, diluents, binders, water-soluble polymers, lubricants, disintegrators, swelling agents, fillers, stabilizers, antioxidants, emulsifiers, emollients, penetration enhancers, propellants, gas, depigmenting agents, film forming agents, gelling agents, moisturizing agents, colorants, fragrance ingredients, exfoliants, solubilizers, solvents, binding agents, bulking agents, humectants, cleansing agents, elastomers, astringents, masking agents, anti-static agents, protectants, denaturants, absorbents, anti-caking agents, matting agents, structuring agents, oxidative agents, reducing agents, superfatting agents, active boosters, and combinations thereof.

Examples of additional agents which may be comprised in the composition include, but are not limited to, desquaming agents, whitening agents, tensing effect agents soothing agents, anti-irritant agents, sebo-regulating agents, wound healing agents, anti-inflammatory agents, anti-acne agents, anti-glycation agents, slimming agents, self-tanning agents, anti-aging agents , anti-wrinkle agents,

Surfactants that can be used in the composition may be anionic, cationic, amphoteric or nonionic. Examples of anionic surfactants include, but are not limited to, carboxylate, sulfonate and sulfate ions-containing surfactants, such as sodium, potassium, ammonium of long chain alkyl sulfonates and alkyl aryl sulfonates such as sodium dodecylbenzene sulfonate; dialkyl sodium sulfosuccinates, such as sodium dodecylbenzene sulfonate; dialkyl sodium sulfosuccinates, such as sodium bis-(2-ethylthioxyl)-sulfosuccinate; sulfated castor oil, propylene glycol, lecithin, capric/caprylic triglycerides, PEG-12 oleate (FANCOL^{®} HS3 US^{®}), and alkyl sulfates such as sodium lauryl sulfate. Cationic surfactants include, but are not limited to, quaternary ammonium compounds such as benzalkonium chloride, benzethonium chloride, cetrimonium bromide, stearyl dimethylbenzyl ammonium chloride, polyoxyethylene and coconut amine. Examples of nonionic surfactants include ethylene glycol monostearate, propylene glycol myristate, glyceryl monostearate, glyceryl stearate, polyglyceryl-4-oleate, sorbitan acylate, sucrose acylate, PEG-150 laurate, PEG-400 monolaurate, polyoxyethylene monolaurate, polysorbates, polyoxyethylene octylphenylether, PEG-1000 cetyl ether, polyoxyethylene tridecyl ether, polypropylene glycol butyl ether, POLOXAMER^{®} 401, stearoyl monoisopropanolamide, polyoxyethylene hydrogenated tallow amide, but also emulsifying wax, glyceryl monooleate, polyoxyethylene alkyl ethers, polyoxyethylene castor oil derivatives, polysorbate, sorbitan esters, benzyl alcohol, benzyl benzoate, cyclodextrins, glycerin monostearate, poloxamer, povidone, cetyl palmitate. Examples of amphoteric surfactants include sodium N-dodecyl-beta-alanine, sodium N-lauryl-beta-iminodipropionate, myristoamphoacetate, lauryl betaine and lauryl sulfobetaine.

Examples of preservatives, include, but are not limited to, benzoic acid, butylparaben, ethyl paraben, methyl paraben, propylparaben, sodium benzoate, sodium propionate, benzalkonium chloride, benzethonium chloride, benzyl alcohol, cetylpyridinium chloride, chlorobutanol, phenol, phenylethyl alcohol, perillic acid and thimerosal.

Examples of water-soluble polymers include, but are not limited to, polyvinylpyrrolidone, dextran, carboxymethylcellulose, and polyethylene glycol.

Suitable stabilizers include, but are not limited to, butylated hydroxytoluene (BHT), ascorbic acid, its salts and esters, Vitamin E, tocopherol and its salts, sulfites such as sodium metabisulphite, cysteine and its derivatives, citric acid, propyl gallate, and butylated hydroxyanisole (BHA).

An example of pH-buffer that can be used in the composition is triethanolamine.

Examples of emollients include, but are not limited to, almond oil, castor oil, ceratonia extract, cetostearoyl alcohol, cetyl alcohol, cetyl esters wax, cholesterol, cottonseed oil, cyclomethicone, ethylene glycol palmitostearate, glycerin, glycerin monostearate, glyceryl monooleate, isopropyl myristate, isopropyl palmitate, lanolin, lecithin, light mineral oil, medium-chain triglycerides, mineral oil and lanolin alcohols, petrolatum, petrolatum and lanolin alcohols, soybean oil, starch, stearyl alcohol, sunflower oil, xylitol and combinations thereof.

Examples of emulsifiers include, but are not limited to, acacia, anionic emulsifying wax, calcium stearate, carbomers, cetostearyl alcohol, cetyl alcohol, cholesterol, diethanolamine, ethylene glycol palmitostearate, glycerin monostearate, glyceryl monooleate, hydroxpropyl cellulose, hypromellose, lanolin, hydrous, lanolin alcohols, lecithin, medium-chain triglycerides, methylcellulose, mineral oil and lanolin alcohols, monobasic sodium phosphate, monoethanolamine, nonionic emulsifying wax, oleic acid, poloxamer, poloxamers, polyoxyethylene alkyl ethers, polyoxyethylene castor oil derivatives, polyoxyethylene sorbitan fatty acid esters, polyoxyethylene stearates, propylene glycol alginate, self-emulsifying glyceryl monostearate, sodium citrate dehydrate, sodium lauryl sulfate, sorbitan esters, stearic acid, sunflower oil, tragacanth, triethanolamine, xanthan gum, PEG-100 Stearate/Glyceryl stearate (Arlacel 165^{®}), decyglucoside (Plantaren 2000^{®}), laurylglucoside (Plantaren1200^{®}), Cetearyl Glucoside, Cetearyl Alcohol (Emulgade PL68/50^{®}), and combinations thereof.

Examples of penetration enhancers include, but are not limited to, fatty alcohols, fatty acid esters, fatty acids, fatty alcohol ethers, amino acids, phospholipids, lecithins, cholate salts, enzymes, amines and amides, complexing agents (liposomes, cyclodextrins, modified celluloses, and diimides), macrocyclics, such as macrocylic lactones, ketones, and anhydrides and cyclic ureas, surfactants, N-methyl pyrrolidones and derivatives thereof, DMSO and related compounds, ionic compounds, azone and related compounds, and solvents, such as alcohols, ketones, amides, polyols (e.g., glycols).

Examples of propellant agents include, but are not limited to, dichlorofluoromethane, difluoroethane, isobutane, n-butane, propane, dichlorofluoromethane, nitrogen, carbon dioxide.

Examples of desquaming agents include, but are not limited to, beta hydroxyacids, alpha hydroxy acids, urea, cinnamic acid, Saphora japonica extract, proteases like trypsine.

Examples of depigmenting agents include, but are not limited to, vitamin C and its derivatives, ferulic acid, resorcinol, alpha and beta arbutin.

Examples of anti-glycation agents include, but are not limited to, black tea extract and Vaccinium myrtillus extract.

Examples of slimming agents include, but are not limited to, caffeine, tea extracts, Hedera helix extracts, and theobromine.

Examples of soothing agents and anti-irritation agents include, but are not limited to, caffeine, vitamins E, C, B5, B3, glycyrrhetic acid, a salt or a derivative thereof.

Examples of sebo-regulating agents include, but are not limited to, zinc salts such as zinc gluconate or zinc pidolate, vitamin B6, selenium chloride, and benzoyl peroxide.

Examples of wound healing agents include, but are not limited to, arginine, hydroxyproline, chitosan and derivatives, propolis extracts, folic acid, and chitosan.

An example of self-tanning agent includes, but is not limited to, erythrulose.

Examples of anti-aging agents include, but are not limited to, placenta extracts, beta glucan, fucoidan, sodium hyaluronate, and collagen.

Examples of anti-static agents includes, but is not limited to, methyl sulfonyl methane.

An example of bulking agent includes, but is not limited to, polypropylene A.

Examples of film former include, but are not limited to, copolymer of 1-vinyl-2-pyrrolidone and vinyl acetate, and Polyquaternium-6.

### Applications

The composition of the invention can be administered orally, topically, parenterally, sub-cutaneously, epicutaneously, intra-dermically, transdermically, intramusculary, enterally, intranasally, intra-respiratory, intra-vascular, ophthalmic preparation, intra-vaginal, endo-urethral, or by nasal inhalation. In a particular embodiment, the composition of the invention is administered sub-cutaneously, epicutaneously, intra-dermically, transdermically, or topically, preferably topically. In a particular embodiment, the composition of the invention is administered by microneedles, or patches.

The composition may in particular be applied to mucosa, corneum, epidermis, dermis, epithelium, endothelium, skin, skin appendages, connective tissues, or bone tissues, preferably skin, skin appendages or mucosa.

In an embodiment, the composition of the invention is selected from a cosmetic composition, a dermatological composition, a therapeutic composition, and a combination thereof.

### Topical Composition

In a particular embodiment, the composition of the invention is a topical composition. The topical composition comprises a set of particles according to the invention, and at least one topically acceptable excipient.

A "topically acceptable excipient", as used herein, denotes an excipient suitable for a topical application. Such an excipient can be judiciously chosen by the skilled artisan, for instance among the excipients described above.

Said topical composition may be a dermatological composition, therapeutic composition and/or a cosmetic composition.

The topical composition may in particular be in the form of a suspension, a cream, a spray, a gel, an aerosol, a butter, a stick, an ointment, a lotion, a solution, a solid, an emulsion, an oil, a lyophilizate, a milk, a powder, a paste, a wax, a mousse or a foam. The composition can be prepared according to processes known to the skilled artisan.

Preferably, the topical composition is selected from a cream, a spray, a gel, an ointment, a lotion, an emulsion, a foam, a suspension and a milk.

The topical composition may be applied to mucosa, corneum, epidermis, dermis, epithelium, endothelium, skin or skin appendages (e.g. hair and nails), preferably mucosa, skin or skin appendages.

### Cosmetic composition

In another particular embodiment, the composition of the invention is a cosmetic composition.

Said cosmetic composition comprises a set of particles according to the invention, and at least one cosmetically acceptable excipient. In such an embodiment, X in particles of formula (I) is advantageously a cosmetic agent.

A "cosmetically acceptable excipient", as used herein, denotes an excipient suitable for a cosmetic application. Such an excipient can be judiciously chosen by the skilled artisan, for instance among the excipients described above.

The cosmetic composition can be administered orally, topically, parenterally, sub-cutaneously, epicutaneously, intra-dermically, transdermically, intramusculary, enterally, intranasally, intra-respiratory, or by nasal inhalation. In a preferred embodiment, the cosmetic composition is administered topically.

Preferably, the cosmetic composition is a topical composition or a dermatological composition, more preferably a topical composition.

The cosmetic composition may in particular be applied to mucosa, corneum, epidermis, dermis, epithelium, endothelium, skin or skin appendages (e.g. hair and nails), preferably mucosa, skin or skin appendages.

The cosmetic composition may in particular be in the form of a suspension, a cream, a spray, an aerosol, a butter, a stick, a gel, an ointment, a lotion, a solution, a solid, an emulsion, an oil, a lyophilizate, a milk, a powder, a paste, a wax, a mousse or a foam. The composition can be prepared according to processes known to the skilled artisan.

The cosmetic composition may in particular be a makeup, skin care, skin styling, skin color, hair care, hair styling or hair color composition.

Examples of makeup include, but are not limited to, primer, setting spray, face powder, eye powder, blush, concealer, highlighter, illuminator, bronzer, liquid or powder foundation, contouring, lip stick, gloss, lip colour, eye liner, lip liner, mascara, eyeshadow, khol, sunless, tanning formulation, nail coat, nail lacquer, or nail polish.

Examples of skin care include, but are not limited to, face masks, cleansers, skin moisturizers, soothers aftershaves, shave gels, shave foams, shower gel, and body gel.

Examples of hair care include, but are not limited to, shampoo, hair moisturizers, or conditioners.

Examples of hair styling include, but are not limited to, hair gel, hair wax, hair foam, hair spray, hair volumizer, or hair pomade.

Examples of hair color include, but are not limited to, dyes.

The cosmetic composition may also be a temporary or permanent tattoo.

The cosmetic composition may also be a fragrance.

The composition of the invention may be particularly well-suited for combatting and/or reducing the signs of cutaneous ageing, such as the formation of wrinkles and/or fine lines, skin sagging, loss of firmness, loss of radiance and/or evenness of the complexion, and/or for reinforcing the skin barrier.

The signs of cutaneous ageing may be related to intrinsic factors that are age-related, but also extrinsic factors, such as UV-light exposure, smoking, and/or pollution.

An object of the invention relates to a cosmetic use of the composition of the invention for combatting and/or reducing the signs of cutaneous ageing, such as the formation of wrinkles and/or fine lines, skin sagging, loss of firmness, loss of radiance and/or evenness of the complexion, and/or for reinforcing the skin barrier.

Another object of the invention is cosmetic process for combatting and/or reducing the signs of cutaneous ageing, such as the formation of wrinkles and/or fine lines, skin sagging, loss of skin firmness, loss of radiance and/or evenness of the complexion, and/or for reinforcing the skin barrier, comprising applying topically to the skin or its appendages, a composition of the invention. In such embodiments, the active agent X may in particular be chosen from skin whitening agents, skin sunless tanning agents, deodorant agents, anti-perspirant agents, antibacterial agents, antifungal agents, sebum reducing agents, anti-acne like keratolytic agents, antiviral agents, antibiotic agents, anti-inflammatory agents, anti-allergic agents, anesthetic agents, anti-pruriginous agents, anti-protozoal agents, antineoplastic agents, immunomodulators, interferons, antidepressants, anti-histamines, vitamins, hormones, antidandruff agents.

### Sunscreen

In another particular embodiment, the composition of the invention is a sunscreen composition.

The sunscreen composition is advantageously applied topically (i.e. a topical composition). The sunscreen composition may in particular be in the form of a suspension, a cream, a spray, an aerosol, a butter, a stick, a gel, an ointment, a lotion, a solution, a solid, an emulsion, an oil, a lyophilizate, a milk, a powder, a paste, a wax, a mousse or a foam. In a particular embodiment, the sunscreen composition is a formulation sunscreen type butter.

In such an embodiment, the active agent X is advantageously an active agent which absorbs and/or reflects UV light. In particular, X may be chosen from UV-filters, photostabilizers, free radical scavengers, and UV filter boosters. Preferably, X is an UV-filter.

### Therapeutic composition

In a particular embodiment, the composition of the invention is a therapeutic composition or a pharmaceutical composition.

The therapeutic composition comprises a set of particles according to the invention and at least one pharmaceutically acceptable excipient. In such an embodiment, the active agent X in particles of formula (I) is advantageously chosen from therapeutic agents.

The term "pharmaceutically acceptable", as used herein, refers to compounds, materials, compositions, and/or dosage forms which are, within the scope of sound medical judgment, suitable for use in contact with the tissues of human beings and animals without excessive toxicity, irritation, allergic response, or other problems or complications commensurate with a reasonable benefit/risk ratio, in accordance with the guidelines of agencies such as the Food and Drug Administration. A "pharmaceutically acceptable excipient", as used herein, refers to all components of a pharmaceutical or therapeutic composition which facilitate the manufacture, the preservation and/or the delivery of the composition *in vivo.* Pharmaceutically acceptable excipients include, but are not limited to, diluents, preservatives, binders, lubricants, disintegrators, swelling agents, fillers, stabilizers, and combinations thereof.

The therapeutic composition can be administered orally, topically, parenterally, sub-cutaneously, epicutaneously, intra-dermically, transdermically, intramusculary, enterally, intranasally, intra-respiratory, or by nasal inhalation. In a preferred embodiment, the therapeutic composition is administered topically.

In a particular embodiment, the therapeutic composition is applied to a tissue chosen from the skin, skin appendages, mucosa, corneum, epidermis, dermis, epithelium, endothelium, connective tissues, bone tissues, and combinations thereof, preferably skin, skin appendages, mucosa, corneum, epidermis, dermis, epithelium, and endothelium, and more preferably skin, skin appendages, and mucosa. In another embodiment, the therapeutic composition is applied to a circulating medium, such as blood or plasma.

In a particular embodiment, the therapeutic composition is a dermatological composition.

Another object of the invention is a set of particles of the invention or a composition of the invention, for use as a drug, a prophylactic vaccine or a therapeutic vaccine.

Another object of the invention is a set of particles of the invention or a composition of the invention (in particular, a therapeutic composition), for use in the treatment and/or prevention of a skin, a mucosa, or an eye cornea disease or condition. Preferably, said skin, mucosa, or eye cornea disease or condition is chosen from lipodystrophy, keloid scars, acne, psoriasis, atopic dermatitis, actinic keratosis, rosacea, melasma, melanoma, Merker cell carcinoma, basal cell carcinoma, squamous cell carcinoma, scar treatments, wound healing, alopecia, vitiligo, urticaria (hives), cold sores, impetigo, eczema, rashes dermatitis, ichthyosis, warts, blisters, pruritus, gangrene, bruises, pustules, bacterial skin infections like leprosy, carbuncles, cellulitis, impetigo, fungal infections like Athlete's foot (intertrigo) and sporotrichosis, fungal nail infections, viral infection like herpes, sunburns, lice, scabies, pressure ulcer disinfection and pressure ulcer healing, vaginitis, bladder cancer, endometriosis, uveitis, cornea diseases, cornea keratitis, corneal herpes, keratoconus, corneal dystrophies, pharyngitis, cutaneous and mucosal allergies.

As used herein, the terms "prevent," "preventing," or "prevention," refer to any reduction, no matter how slight, of a subject's predisposition or risk for developing a condition, disease, disorder or symptom thereof. For purposes of prevention, the subject is any subject, and preferably is a subject that is at risk for, or is predisposed to, developing a condition, disease, disorder. The term "prevention" includes either preventing the onset of a clinically evident condition, disease, disorder altogether or preventing the onset of a pre-clinically evident condition, disease, disorder in individuals at risk. This includes prophylactic treatment of subjects at risk of developing condition, disease, disorder.

As used herein, the terms "treat", "treatment" or "treating" of a disease, disorder, or condition encompasses alleviation of at least one symptom thereof, a reduction in the severity thereof, or the delay or inhibition of the progression thereof. Treatment need not mean that the disease, disorder, or condition is totally cured. A useful composition herein needs only to reduce the severity of a disease, disorder, or condition, reduce the severity of symptoms associated therewith, provide improvement to a patient or subject's quality of life, or delay or inhibit the onset of a disease, disorder, or condition.

Another object of the invention is a method for delivering at least one active agent X to a tissue or a circulating medium (e.g. blood, plasma), preferably a tissue, of a subject in need thereof, comprising administering an effective amount of a composition of the invention. The present invention also provides a method of delivering at least one active agent X to a tissue of a subject, comprising: topically administering to a subject in need thereof a therapeutically effective amount of any presently described compositions useful in treating a disease, disorder, or condition of the tissue. Particularly, said tissue is chosen from the skin, skin appendages, corneum, epidermis, dermis, epithelium, endothelium, connective tissues, bone tissues, and combinations thereof. Preferably, said tissue is the skin, a skin appendage, or a mucosa.

"Effective amount" or "therapeutically effective amount", as used herein, refers to an amount of drug or composition of the invention as disclosed herein effective to alleviate, delay onset of, or prevent one or more symptoms of a disease or disorder.

Another object of the invention is a method for treating or preventing a skin, a mucosa, or an eye cornea disease or condition, comprising administering to a subject in need thereof, a composition of the invention (in particular, a therapeutic composition), said composition comprising at least one bioadhesive particle of formula (I) as defined herein. In a preferred embodiment, X is a therapeutic agent suitable for treating or preventing a skin, a mucosa, or an eye cornea disease or condition. A further object of the invention is a method of treating or preventing a skin, a mucosa, or an eye cornea disease, disorder, or condition in a subject, comprising: topically administering to a subject in need thereof a therapeutically effective amount of any presently described compositions useful in treating a skin, a mucosa, or an eye cornea disease, disorder, or condition. In a particular embodiment, the skin, mucosa, or eye cornea disease, disorder, or condition is selected from lipodystrophy, keloid scars, acne, psoriasis, atopic dermatitis, actinic keratosis, rosacea, melasma, melanoma, Merker cell carcinoma, basal cell carcinoma, squamous cell carcinoma, scar treatments, wound healing, alopecia, vitiligo, urticaria (hives), cold sores, impetigo, eczema, rashes dermatitis, ichthyosis, warts, blisters, pruritus, gangrene, bruises, pustules, bacterial skin infections like leprosy, carbuncles, cellulitis, impetigo, fungal infections like Athlete's foot (intertrigo) and sporotrichosis, fungal nail infections, viral infection like herpes, sunburns, lice, scabies, pressure ulcer disinfection and pressure ulcer healing, vaginitis, bladder cancer, endometriosis, uveitis, cornea diseases, cornea keratitis, corneal herpes, keratoconus, corneal dystrophies, pharyngitis, cutaneous and mucosal allergies.

For the purpose of clarity, any element or feature of any method or composition or process described herein, can be combined with any other element or feature of any other method or composition or process described herein.

Another object of the invention is a use of a set of particles of the invention for making a composition for treating and/or preventing a skin, mucosa, eye cornea disease or condition.

Another object of the invention is a set of particles of the invention or a composition of the invention (in particular, a therapeutic composition), for use in the treatment and/or prevention of a disease or condition selected in the group consisting of lipodystrophy, keloid scars, acne, psoriasis, atopic dermatitis, actinic keratosis, rosacea, melasma, melanoma, Merker cell carcinoma, basal cell carcinoma, squamous cell carcinoma, scar treatments, wound healing, alopecia, vitiligo, urticaria (hives), cold sores, impetigo, eczema, rashes dermatitis, ichthyosis, warts, blisters, pruritus, gangrene, bruises, pustules, bacterial skin infections like leprosy, carbuncles, cellulitis, impetigo, fungal infections like Athlete's foot (intertrigo) and sporotrichosis, fungal nail infections, viral infection like herpes, sunburns, lice, scabies, pressure ulcer disinfection and pressure ulcer healing, vaginitis, cancer such as bladder cancer, endometriosis, uveitis, cornea diseases, cornea keratitis, corneal herpes, keratoconus, corneal dystrophies, pharyngitis, cutaneous and mucosal allergies.

A further object of the invention is a set of particles of the invention or a composition of the invention, for use in the treatment and/or prevention of cancer, aneurysm embolization, angioma, tumors, thrombus, preferably by magnetic guidance. In a particular embodiment, the set of particles comprises particles wherein X is a therapeutic agent and particles wherein X is a magnetic agent or a magnetic molecule. In a further particular embodiment, the set of particles comprises particles wherein one of X is a therapeutic agent and the other X is a magnetic agent or a magnetic molecule.

A further object of the invention is a set of particles of the invention or a composition of the invention, for use in the treatment and/or prevention of an allergy, particularly a food allergy, such as peanut or a milk/lactose allergy.

A further object of the invention is a set of particles of the invention or a composition of the invention, for use for controlling parasites. As used herein, the expressions "controlling parasites" and "control of parasites" comprise the reduction, the elimination, the killing, the prevention and/or the treatment of parasites in a subject. Examples of parasites includes for instance ectoparasites and endoparasites, such as insects, ticks, flea, phlebotomes, mites, and worms.

### Medical device

Another object of the invention is a medical device comprising a set of particles according to the invention.

In a particular embodiment, said medical device is selected from an implant, plaster, wound dressing, patch and lens. The medical device can be coated or grafted with the set of particles of the invention.

An "implant", as used herein, refers to a device or element that is structured, sized, or otherwise configured to be implanted, preferably by injection or surgical implantation, in a specific region of the body so as to provide therapeutic benefit by releasing one or more active agents over an extended period of time at the site of implantation.

### Kit

The present invention also relates to a kit comprising:
- a composition according to the invention,
- a washing composition, and
- optionally an instruction guide.

In a preferred embodiment, the composition according to the invention in the kit is a topical composition. In a more preferred embodiment, the composition according to the invention in the kit is a cosmetic or a sunscreen composition, preferably a sunscreen composition.

A "washing composition" refers to a composition which enables the removal of part or all of a composition according to the invention, previously applied to a tissue such as the skin, skin appendages or a mucosa of a subject. More specifically, the washing composition enables the removal of bioadhesive particles which adhere to the tissue.

The washing composition may comprise at least one "washing agent" and, optionally one or more excipients. The "washing agent" refers to a chemical or biological agent which is able to break the bond between bioadhesive particles adhered to a tissue and said tissue. The washing agent may be a protein, a peptide (e.g. glutathione), an amino acid, an enzyme (e.g. cathepsin B), a thiol (e.g. 2-mercaptoethanol, N-acetyl cysteine), a dithiol (e.g. dithiothreitol), a pH-modifier, an acid, a base, a solvent, a saline solution (e.g. sodium chloride solution) or a combination thereof. Said washing agent can be judiciously chosen by the skilled artisan, depending on the nature of the bond between bioadhesive particles adhered to a tissue and said tissue.

In a particular embodiment, said washing composition is a powder, a shampoo, a soap, a lotion, a solution, a solid, a scrubbing, a scraper, a mousse, a foam, a syndet, a gel, a shower gel, a spray, a mist, a wax, a strip, an enzyme composition, a detergent composition or a woven or non-woven fabric.

The invention will also be described in further detail in the following examples, which are not intended to limit the scope of this invention, as defined by the attached claims.

### EXAMPLES

| Polymer | [B-C] moiety | UV filter | UV filter logP |
|---|---|---|---|
| PMMA (Eudragit L100) | PEG-S-S-(2-pyridyldithio) | Octocrylene (OCR) | 7.1 |
| PMMA (Eudragit RSPO) | Without linker | Bemotrizinol (BEMT) | 10.4 |
| Cellulose (Natrosol 250) | Maleimide-(CH₂)₁₀-COOH | Octyltriazone (EHT) | 14.5 |
| PLA (Gotalene RS41 MW: 100 K) | PEG-maleimide | Bemotrizinol | |
| Cellulose acetate | ε-thiocaprolactone | Octisalate (EHS) | 5.7 |
| Chitosan (Chitosan, HCl) | Maleimide-PEG₈-succinimidyl ester | Meradimate (MA) | 5.1 |
| Chitosan (Chitosan, HCl) | Maleimide-(CH₂)₁₀-COOH | Sulisobenzone (BP4) | 2.2 |
| PLA | PEG-Mal | Uvinul A Plus | 6.5 |
| PLGA | PEG-VS | Uvinul A Plus | 6.5 |
| PLGA | PEG-VS | Octocrylene | 7.1 |
| PLA | -O-CH₂-CH₂-Mal | Uvinul A Plus | 6.5 |
| PLA | mPEG(methoxy-polyethylene glycol) | Uvinul A Plus | 6.5 |

The synthesis of amphiphilic poly(ethylene glycol) (PEG) diblock copolymers with functional groups at the PEG chain ends was adapted from the procedures described by Xiaohan Wu and Suming Li (Polym Int, 6(7), 1014-1021, 2012).

### Example 1. Preparation of particles of Polymethacrylate - PEG-OPSS

### Eudragit L100-PEG-OPSS

8.9g of Eudragit L100 (Mn=125 000) and 1g OPSS-PEG-NH2 (Mw=3500) were suspended in DMF. 0.013 mL of diisopropylcarbodiimide (DIC) and 10 mg of DMAP were added and the reaction mixture was stirred for several days at room temperature under inert atmosphere. DMF was concentrated to dryness and the residue triturated with a mixture of acetone and methanol and centrifuged. The supernatant was eliminated and the residue was washed with a large amount of methanol to remove uncoupled PEG. The precipitate obtained was collected by filtration and vacuum dried to yield Eudragit L100-PEG-OPSS as an amorphous solid.

### • Preparation of the polymer solution

10 grams of Eudragit L100-PEG-OPSS were added under stirring into 100 grams of ethyl acetate in a vessel at room temperature for 5 minutes, heating to 50°C and dissolved under stirring for about 20 minutes to homogenous solution.

The polymer solution was cooled to room temperature and 7 grams octocrylene (BASF, Uvinul^{®} N 539 T) dissolved in 30 grams of DMSO, were added to the polymer solution under stirring for about 8 minutes.

### • Preparation of the emulsion and manufacture of bioadhesive nanoparticle

An aqueous solution was prepared by mixing 200 grams of water with 2% w/w of PVA Mowiol 4-88.

20 grams of ethyl acetate was added to the water phase and the polymer solution was then gradually added into the water with the help of an Ultra Turax (Ystral) at 16000 tr/min for 5 minutes.

Emulsion droplet sizes were reduced by sonication conducted with a sonication probe at 100% amplitude for 60 seconds at room temperature.

The fine emulsion is immediately diluted in 500 grams of water.

The solvents were evaporated with a rotating evaporator. The solution was centrifuged and freeze dried to obtain the nanoparticles.

### Example 2. Preparation of particles of Eudragit RSPO^{®} ammonium methacrylate copolymer type B (poly(ethyl acrylate)-co-(methyl methacrylate)-co-(trimethylammonium-ethyl methacrylate chloride)

### Eudragit RSPO^{®}

### • Preparation of the polymer solution

10 grams of Eudragit RSPO were added under stirring into 100 grams of ethyl acetate in a vessel at room temperature for 5 minutes, heating to 50°C and dissolved under stirring for about 20 minutes to homogenous solution.

The polymer solution was cooled to room temperature and 10 grams of Bemotrizinol (BASF, Tinosorb S) dissolved in 30 grams of tetrahydrofuran, were added to the polymer solution under stirring for about 8 minutes.

### • Preparation of the emulsion and manufacture of bioadhesive nanoparticle

An aqueous solution was prepared by mixing 200 grams of water with 2% w/w of PVA Mowiol 4-88.

20 grams of ethyl acetate was added to the water phase and the polymer solution was then gradually added into the water with the help of an Ultra Turax (Ystral) at 16000 tr/min for 5 minutes.

Emulsion droplet sizes were reduced by sonication conducted with a sonication probe at 100% amplitude for 60 seconds at room temperature.

The fine emulsion is immediately diluted in 500 grams of water.

The solvents were evaporated with a rotating evaporator. The solution was centrifuged and freeze dried to obtain the nanoparticles.

### Example 3. Preparation of particles of hydroxyethylcellulose-O-C(O)-(CH₂)₁₀-maleimide

### Hydroxyethylcellulose -OC(O)-(CH₂)₁₀-maleimide

Natrosol 250 (50 g) was dissolved in 2500 mL of fresh LiCl/DMAc solvent (9% wt/wt) at 90°C under mechanical stirring for 30 min. A mixture of Maleimide-(CH₂)₁₀-COOH (0.93 g), acetic anhydride (0.32 mL), and HClO4 catalyst (0.002 mL) was stirred for 1 h at 90°C. The hot mixture was poured into a previously prepared natrosol solution. The whole was then refluxed under mechanical stirring for several hours at 130°C. The esterified hydroxyethylcellulose was recovered at the end of the reaction by precipitation with a large volume of 50% aqueous ethanol. The suspension was vacuum-filtered over fritted glass, thoroughly washed with ethanol and with deionized water and was dried at 105°C to constant weight and stored in a dessicator at room temperature.

### • Preparation of the polymer solution

10 grams of hydroxyethylcellulose -OC(O)-(CH₂)₁₀-maleimide were added under stirring into 100 grams tetrahydrofuran in a vessel at room temperature for 5 minutes, heating to 35°C and dissolved under stirring for about 20 minutes to homogenous solution.

The polymer solution was cooled to room temperature and 10 grams octyltriazone (BASF, Uvinul^{®} T150) dissolved in 30 grams of tetrahydrofuran, were added to the polymer solution under stirring for about 8 minutes.

### • Preparation of the emulsion and manufacture of bioadhesive nanoparticle

An aqueous solution was prepared by mixing 200 grams of water with 2% w/w of PVA Mowiol 4-88.

20 grams of ethyl acetate was added to the water phase and the polymer solution was then gradually added into the water with the help of an Ultra Turax (Ystral) at 16000 tr/min for 5 minutes.

Emulsion droplet sizes were reduced by sonication conducted with a sonication probe at 100% amplitude for 60 seconds at room temperature.

The fine emulsion is immediately diluted in 500 grams of water.

The solvents were evaporated with a rotating evaporator. The solution was centrifuged and freeze dried to obtain the nanoparticles.

### Example 4. Preparation of particles of Polylactic acid -PEG Maleimide

### Polylactic acid Gotalene RS411 - PEG Maleimide

To a solution of 15g Gotalene RS411 (Mw=96500 Da, Mn=77000 Da) and 4.8g Maleimide-PEG5K-OH in dry DCM were added 48 mg of N-(3-Dimethylaminopropyl)-N'-ethylcarbodiimide hydrochloride (EDC-HCl) and 10 mg of DMAP. The reaction mixture was stirred for 2 days at room temperature in an argon atmosphere. Then the solution was concentrated to dryness and the residue triturated several times with methanol to remove traces of unreacted PEG. The precipitate was collected by filtration and vacuum dried to yield PLA-PEG-Maleimide.

### • Preparation of the polymer solution

10 grams of polylactic acid Gotalene RS411 - PEG Maleimide were added under stirring into 200 grams dichloromethane in a vessel at room temperature for 5 minutes and dissolved under stirring for about 20 minutes to homogenous solution.

The polymer solution was cooled to room temperature and 10 grams Bemotrizinol (BASF, Tinosorb S) dissolved in 30 grams of tetrahydrofuran, were added to the polymer solution under stirring for about 8 minutes.

### • Preparation of the emulsion and manufacture of bioadhesive nanoparticle

An aqueous solution was prepared by mixing 200 grams of water with 2% w/w of PVA Mowiol 4-88.

20 grams of ethyl acetate was added to the water phase and the polymer solution was then gradually added into the water with the help of an Ultra Turax (Ystral) at 16000 tr/min for 5 minutes.

Emulsion droplet sizes were reduced by sonication conducted with a sonication probe at 100% amplitude for 60 seconds at room temperature.

The fine emulsion is immediately diluted in 500 grams of water.

The solvents were evaporated with a rotating evaporator. The solution was centrifuged and freeze dried to obtain the nanoparticles.

### Example 5. Preparation of particles of Cellulose acetate- ε-thiocaprolactone

### Cellulose acetate- ε-thiocaprolactone

A mixture of 2.5 g cellulose acetate, 10 mL thiocaprolactone (prepared according to Cüneyt H. Ünlü et al. Int. J. Mol. Sci. 2018, 19, 3799) and 0.1 wt% catalyst Tin(II) 2-ethylhexanoate was reacted at 180°C using a thermostatic oil bath for 15 min under reduced pressure and continuous nitrogen gas purging. Then composites were then further purified by dissolving the polymer products with a small amount of toluene and then precipitating them in hot methanol to remove the unreacted thiocaprolactone and short chain polythiocaprolactone. The extracted sample was dried in a vacuum at 60°C for 24 hours.

### • Preparation of the polymer solution

10 grams of cellulose acetate- thiocaprolactone were added under stirring into 200 grams dimethylsulfoxide in a vessel at room temperature for 5 minutes and dissolved under stirring for about 20 minutes to homogenous solution.

The polymer solution was cooled to room temperature and 5 grams Octisalate (BASF, Tinosorb S) dissolved in 50 grams of dimethylsulfoxide, were added to the polymer solution under stirring for about 10 minutes.

### • Preparation of the emulsion and manufacture of bioadhesive nanoparticle

An aqueous solution was prepared by mixing 200 grams of water with 2 % w/w of Polysorbate 80.

20 grams of dimethylsulfoxide was added to the water phase and the polymer solution was then gradually added into the water with the help of an Ultra Turax (Ystral) at 16000 tr/min for 5 minutes.

Emulsion droplet sizes were reduced by sonication conducted with a sonication probe at 100% amplitude for 60 seconds at room temperature.

The fine emulsion is immediately diluted in 500 grams of water.

The solvents were evaporated with a rotating evaporator. The solution was centrifuged and freeze dried to obtain the nanoparticles.

### Example 6. Preparation of particles of Chitosan- PEG₂₄-Maleimide

### Chitosan- PEG₂₄-Maleimide

25 mg Chitosan and 10 mg Maleimide-PEG₂₄-succinimidyl ester were dissolved in H2O (5 mL). Then, 35 mg EDC-HCl were added in four portions every 30 min. The resulting solution was stirred at room temperature overnight and then purified by size exclusion chromatography and lyophilized to afford a white foam.

### • Preparation of the polymer solution

10 grams of Chitosan- PEG₂₄-Maleimide were added under stirring into 200 grams dimethylsulfoxide in a vessel at room temperature for 5 minutes and dissolved under stirring for about 20 minutes to homogenous solution.

The polymer solution was cooled to room temperature and 5 grams Meradimate dissolved in 50 grams of dimethylsulfoxide, were added to the polymer solution under stirring for about 10 minutes.

### • Preparation of the emulsion and manufacture of bioadhesive nanoparticle

200 grams of ethanol was prepared with 2 % w/w of Polysorbate 80.

20 grams of dimethylsulfoxide was added to the ethanol solution and the polymer solution was then gradually added into the water with the help of an Ultra Turax (Ystral) at 16000 tr/min for 5 minutes.

Emulsion droplet sizes were reduced by sonication conducted with a sonication probe at 100% amplitude for 60 seconds at room temperature.

The fine emulsion is immediately diluted in 500 grams of ethanol.

The solvents were evaporated with a rotating evaporator. The solution was centrifuged and freeze dried to obtain the nanoparticles.

### Example 7. Preparation of particles of Chitosan-NH-CO-(CH₂)₁₀-maleimide

To a suspension of 25 mg Chitosan, 6 mg Maleimide-(CH₂)₁₀-COOH and 100 µL of a 16.8 mg/mL solution of N-hydroxysuccinimide (NHS) in a mixture of THF (2.5 mL) and H2O (2.5 mL) were added 35 mg EDC-HCl in four portions every 30 min. The resulting solution was stirred at room temperature for 24h, then purified by size exclusion chromatography and lyophilized to afford a white foam.

### • Preparation of the polymer solution

10 grams of Chitosan-NH-CO-(CH₂)₁₀-maleimide were added under stirring into 200 grams a buffer in a vessel at room temperature for 5 minutes and dissolved under stirring for about 20 minutes to homogenous solution.

The polymer solution was cooled to room temperature and 7 grams Sulisobenzone dissolved in 50 grams of buffer, were added to the polymer solution under stirring for about 10 minutes.

### • Preparation of the emulsion and manufacture of bioadhesive nanoparticle

200 grams of toluene was prepared with 2 % w/w of Polysorbate 80.

20 grams of buffer was added to the toluene solution and the polymer solution was then gradually added into the water with the help of an Ultra Turax (Ystral) at 16000 tr/min for 5 minutes.

Emulsion droplet sizes were reduced by sonication conducted with a sonication probe at 100% amplitude for 60 seconds at room temperature.

The fine emulsion is immediately diluted in 500 grams of toluene.

The solvents were evaporated with a rotating evaporator. The solution was centrifuged and freeze dried to obtain the nanoparticles.

### Example 8. Preparation of particles of Poly DL lactic acid (PLA)- Polyethylene glycol (PEG)-Maleimide (Mal) (PLA-PEG-Mal) with Uvinul A Plus - Batch 2

### • Preparation of the polymer solution

150 mg of PLA 10 KDa-PEG 1 KDa- Mal (purchased at Nanosoft Polymers (USA)) were solubilized in 4.8 mL of acetone. 50 mg of Uvinul A Plus (BASF) were added and the mixture was mixed until complete dissolution at room temperature.

### • Preparation of the emulsion and manufacture of bioadhesive nanoparticles

A solution of 2% w/w poly vinyl alcohol (Mw 31 000) in water was prepared. 8 grams of the PVA solution was added to the polymer solution under stirring. The emulsion droplets were reduced by sonication conducted by a sonication probe at 100 % amplitude for 3 seconds at room temperature.

The fine emulsion was immediately diluted into 20 mL of water and vigorously mixed for 30 seconds.

The solvent was evaporated with a rotating evaporator. The suspension was centrifuged at 3000 rpm in Amicon 100 KDa centrifugation tubes. The centrifuged solution containing the particles was resuspended into water and stored in the fridge.

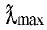 UV absorbance of free Uvinul A plus in ethanol was measured at 357 nm and 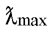 UV absorbance of Uvinul A plus encapsulated PLA-PEG-Mal particles in water was measured at 360 nm.

A UV-filter encapsulation rate of 30%(w/w) was determined by HPLC after treatment of particles in an acetic solution. UV-filter encapsulation rates of 40% (Batch 3) and 47% (Batch 4) were obtained with two other batches of PLA-PEG-Mal with Uvinul A Plus.

### Example 9. Preparation of particles of Poly(L-lactide-co-glycolide)-b-poly(ethylene glycol)-vinylsulfone (PLGA-PEG-VS) with Uvinul A Plus- Batch 5

### • Preparation of the polymer solution

155 mg of PLGA 10KDa-PEG 2KDa-VS (purchased at Nanosoft Polymers (USA)) were solubilized in 4.8 mL of acetone. 50 mg of Uvinul A Plus (BASF) were added and the mixture is mixed until complete dissolution at room temperature.

### • Preparation of the emulsion and manufacture of bioadhesive nanoparticles

A solution of 2% w/w poly vinyl alcohol (Mw 31 000) in water was prepared. 8 grams of the PVA solution was added to the polymer solution under stirring. The emulsion droplets were reduced by sonication conducted by a sonication probe at 100 % amplitude for 3 seconds at room temperature.

The fine emulsion was immediately diluted into 20 mL of water and vigorously mixed for 30 seconds.

The solvent was evaporated with a rotating evaporator. The suspension was centrifuged at 3000 rpm in Amicon 100 KDa centrifugation tubes. The centrifuged solution containing the particles was resuspended into water and stored in the fridge.

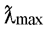 UV absorbance of free Uvinul A plus in ethanol and Uvinul A plus encapsulated PLGA-PEG-VS particles in water were both measured at 357 nm.A UV-filter encapsulation rate of 4.7%(w/w) was determined by HPLC after treatment of particles in an acetic solution.

### Example 10. Preparation of particles of Poly(L-lactide-co-glycolide)-b-poly(ethylene glycol)-vinylsulfone (PLGA-PEG-VS) with Octocrylene - Batch 6

### • Preparation of the polymer solution

155 mg of PLGA 10KDa-PEG 2KDa-VS (purchased at Nanosoft Polymers (USA)) were solubilized in 4.8 mL of acetone. 50 mg of Octocrylene (BASF) were added and the mixture was mixed until complete dissolution at room temperature.

### • Preparation of the emulsion and manufacture of bioadhesive nanoparticles

A solution of 2% w/w poly vinyl alcohol (Mw 31 000) in water was prepared. 8 grams of the PVA solution was added to the polymer solution under stirring. The emulsion droplets were reduced by sonication conducted by a sonication probe at 100 % amplitude for 3 seconds at room temperature.

The fine emulsion was immediately diluted into 20 mL of water and vigorously mixed for 30 seconds.

The solvent was evaporated with a rotating evaporator. The suspension was centrifuged at 3000 rpm in Amicon 100 KDa centrifugation tubes. The centrifuged solution containing the particles was resuspended into water and stored in the fridge.

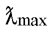 UV absorbance of free Octocrylene in ethanol was measured at 304 nm and 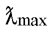 UV absorbance of Octocrylene encapsulated PLGA-PEG-VS particles in water was measured at 360 nm.

A UV-filter encapsulation rate of 74.2%(w/w) was determined by HPLC after treatment of particles in an acetic solution.

### Example 11. Preparation of particles of Poly(L-lactide) N-2-hydroxyethylmaleimide (PLA-O-CH₂-CH₂-Mal) with Uvinul A Plus - Batch 7

### • Preparation of the polymer solution

150 mg of PLA-O-CH₂-CH₂-Mal (purchased at Sigma Aldrich) were solubilized in 8 mL of dichloromethane. 50 mg of Uvinul A Plus (BASF) were added and the mixture was mixed until complete dissolution at room temperature.

### • Preparation of the emulsion and manufacture of bioadhesive nanoparticles

A solution of 2% w/w poly vinyl alcohol (Mw 31 000) in water was prepared. 8 grams of the PVA solution was added to the polymer solution under stirring. The emulsion droplets were reduced by sonication conducted by a sonication probe at 100 % amplitude for 3 seconds at room temperature.

The fine emulsion was immediately diluted into 20 mL of water and vigorously mixed for 30 seconds.

The solvent was evaporated with a rotating evaporator. The suspension was centrifuged at 3000 rpm in Amicon 100 KDa centrifugation tubes . The centrifugated solution containing the particles was resuspended into water and stored in the fridge.

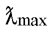 UV absorbance of free Uvinul A plus in ethanol was measured at 357 nm and 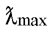 UV absorbance of Uvinul A plus encapsulated PLA-O-CH₂-CH₂-Mal particles in water was measured at 358 nm.

A UV-filter encapsulation rate of 3.3%(w/w) was determined by HPLC after treatment of particles in an acetic solution.

### Example 12. Preparation of particles of Methoxy poly(ethylene glycol)- poly (D,L Lactide) (PLA-mPEG) - Batch 1

### • Preparation of the polymer solution

200 mg of mPEG 15KDa-PLA 15KDa (purchased at Sigma Aldrich) were solubilized in 4.8 mL of acetone. 50 mg of Uvinul A Plus (BASF) were added and the mixture is mixed until complete dissolution at room temperature.

### • Preparation of the emulsion and manufacture of bioadhesive nanoparticles

A solution of 2% w/w poly vinyl alcohol (Mw 31 000) in water was prepared. 8 grams of the PVA solution was added to the polymer solution under stirring. The emulsion droplets were reduced by sonication conducted by a sonication probe at 100 % amplitude for 3 seconds at room temperature.

The fine emulsion was immediately diluted into 20 mL of water and vigorously mixed for 30 seconds.

The solvent was evaporated with a rotating evaporator. The suspension was centrifuged at 3000 rpm in Amicon 100 KDa centrifugation tubes. The centrifuged solution containing the particles was resuspended into water and stored in the fridge.

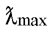 UV absorbance of free Uvinul A plus in ethanol was measured at 357 nm and 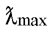 UV absorbance of Uvinul A plus encapsulated PLA-mPEG particles in water was measured at 354 nm.

A UV-filter encapsulation rate of 25%(w/w) was determined by HPLC after treatment of particles in an acetic solution.

### Example 13. Bioadhesion Tests

The particle bio adhesion was carried out on a collagen matrix, for protein binding, and on a cysteine-collagen matrix, for specific binding of sulfhydryl-reactive chemical groups. The bio adhesion test is based on the presence of UV filter encapsulated into the particles and assays the absorbance of the particles after bio adhesion.

UV-star 96-well plates (Greiner Bio-One ref 655801) were coated by incubating 50 µL/well of a collagen (20 µg/mL, Sigma C7661) solution or a collagen (20 µg/mL) and cysteine (2 mg/mL, Wacker 60112636) solution for 2 hours at room temperature on slight agitation (200 rpm). Water was used as a negative control (no matrix coating). After removing coating solutions in excess, wells were washed 3 times with 100 µL/well water and dried at room temperature for 16 hours.

For bio adhesion, 100 µL/well of particles were incubated for 1 hour at room temperature on slight agitation (200 rpm). Absorbance at the specific UV filter wavelength was read using Omega FLUOstar plate reader (BMG Labtech) over the well bottom surface after 11 water washes (pH7). Each sample was run on three wells and the average of absorbance was calculated for each well.

Results are shown in Table 1 below.

**Table 1**

| Batch | Particles | Collagen matrix | Cysteine-collagen-matrix | Water | Well n = | Wash number n = |
|---|---|---|---|---|---|---|
| 1 | PLA-mPEG + Uvinul A Plus | 0.09 | 0.1 | 0.07 | 3 | 11 |
| 2 | PLA-PEG-Mal +Uvinul A Plus | 0.17 | 0.31 | 0.044 | 3 | 11 |
| 3 | PLA-PEG-Mal +Uvinul A Plus | 0.13 | 0.23 | 0.08 | 3 | 11 |
| 4 | PLA-PEG-Mal +Uvinul A Plus | 0.11 | 0.24 | 0.09 | 3 | 11 |
| 5 | PLGA-PEG-VS + Uvinul A Plus | 0.18 | 0.21 | 0.09 | 3 | 11 |
| 6 | **PLGA-PEG-VS** + Octocrylene | 0.15 | 0.18 | 0.07 | 3 | 11 |
| 7 | PLA-O-CH₂-CH₂-Mal + Uvinul A Plus | 0.12 | 0.21 | 0.06 | 3 | 11 |

Bio-adhesion was demonstrated with particles of the invention, on collagen matrix and in particular on cysteine-collagen-matrix. Efficient bio-adhesions were obtained regardless of the nature of the encapsulated UV-filter (Uvinul A Plus and Octocrylene).

More particularly, a high bio-adhesion on collagen matrix and on cysteine-collagen-matrix was obtained with PLGA-PEG-VS, PLA-PEG-Mal, and PLA-O-CH₂-CH₂-Mal (Batches 2-7).

### Example 14. Particles in vitro skin adhesion tests

A synthetic skin-like polymer substrate VITRO SKIN^{®} with similar properties to human (topography, surface tension, ionic strength, pH) is used. The surface of the VITRO SKIN^{®} is coated with poly-L-lysine (PLL) to provide amine density similar to protein rich stratum corneum. The particles are loaded with rhodamine 123. Particle adhesion is measured by washing the substrate with water and measuring the water resistance of bound particles on washed and unwashed substrates using fluorescence microscopy.

Serum albumin bovine (BSA) is coated on a substrate. The bioadhesive particles are loaded with rhodamine 123. Particle adhesion is measured versus control by fluorescence microscopy after washing the BSA coated substrate with an aqueous physiological buffer.

## Claims

1. A set of particles comprising at least 20% by weight of bioadhesive particles, relative to the total weight of the set, said bioadhesive particles are represented by the following formula (I):
{A-[(B)ₙ-C]ₘ} (I),
in which:
- A represents a polymer core,
- B represents a linear linker,
- C represents a functional group chosen from an aldehyde, an acetal, thiocetal, a thiol, a maleimide, a Mickael acceptor, a vinylsulfone, a sulfonylaziridine, an epoxide, a haloacetyl, an isocyanate, an isothiocyanate, a N-hydroxysuccinimide ester, a N-hydroxysulfosuccinimide ester, a hydroxy, an amino, an ammonium, a guanidinium, a carboxylic acid, a carboxylic ester, an anhydride, a sulfonic acid, folic acid, biotin, streptavidin, avidin, antibodies, single chain antibodies or fragments thereof,
- n is 1, and
- m is an integer from 1 to 10²⁵, preferably from 1 to 2000, and
where said bioadhesive particle encapsulates or is attached to at least one active agent X, which is a UV-filter.

2. The set of particles according to claim 1, wherein:
- A comprises at least one residue of a polymer chosen from poly(lactic acid), poly(glycolic acid), poly(lactic co glycolic), polyacrylic acid, acrylic acid copolymers, poly(methacrylic acid), polyalkylacrylates (e.g. polymethacrylates, polybutyl acrylates), polyalkylcyanoacrylates, polyvinylacetate phtalate, cellulose, ethylcellulose, cellulose acetate, cellulose acetate phthalate, hydroxyethylcellulose, carboxymethylcellulose, polyvinyl alcohol, polycaprolactone, polycaprolactone crosspolymers, styrene copolymers, nylons, polymethylsilsesquioxane, poly(2-ethyl-2-oxazoline), povidone, alginate, dextran, chitosan, poly(methylvinylether/maleic anhydride) copolymers, poly(lactic acid)-b-poly(ethyleneglycol), and combinations thereof.

3. The set of particles according to claim 1 or 2, wherein B comprises at least one residue of a polymer chosen from polyalkylene glycols such as polyethylene glycol, poly(methacrylic acid), polymethacrylates, polyalkylcyanoacrylates, polyvinylacetate phtalate, polyvinyl alcohol, povidone, and poly(methylvinylether/maleic anhydride) copolymers, and combination thereof.

4. The set of particles according to any one of claims 1 to 3, wherein B is a group of formula (II):
-[Y-(CH₂)_{q}]ₖ-(O-CH₂-CH₂)ₚ- (II),
in which:
- Y is selected from -O-, -NH-, and -C(O)-,
- p is an integer from 0 to 25,
- k is 0 or 1,
- q is an integer from 1 to 10, and
- p+k is different from 0.

5. The set of particles according to any one of claims 1 to 4, wherein C is chosen from a thiol, a maleimide, a Mickael acceptor, a vinylsulfone, a sulfonylaziridine, an epoxide, a haloacetyl, an isocyanate, an isothiocyanate, a N-hydroxysuccinimide ester, a N-hydroxysulfosuccinimide ester, a hydroxy, an amino, a guanidinium, an ammonium, a carboxylic acid, a carboxylic ester, an anhydride, a sulfonic acid, folic acid, biotin, streptavidin, avidin, antibodies, single chain antibodies or fragments thereof.

6. The set of particles according to any one of claims 1 to 5, wherein:
- A comprises at least one residue of a polymer chosen from poly(lactic acid) and poly(lactic co glycolic);
- B comprises at least one residue of poly(ethylene glycol), or B is a group of formula (II) as defined in claim 4, wherein k is 0 and p is 1;
- C is a maleimide of formula (Mal 1), and (Mal 2): 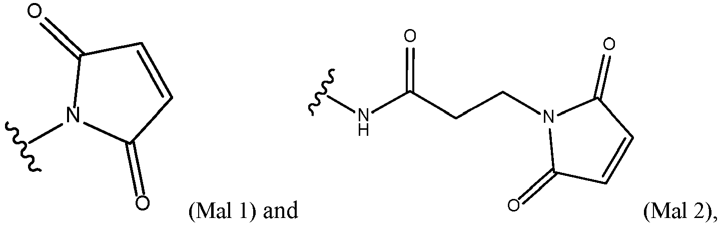 or a vinyl sulfone; and
- m is comprised between 1 and 10²⁵, preferably between 1 and 2000.

7. The set of particles according to any one of claims 1 to 6, wherein:
- A comprises at least one residue of poly(lactic acid), B comprises at least one residue of poly(ethylene glycol), C is maleimide of formula (Mal 1), and (Mal 2): 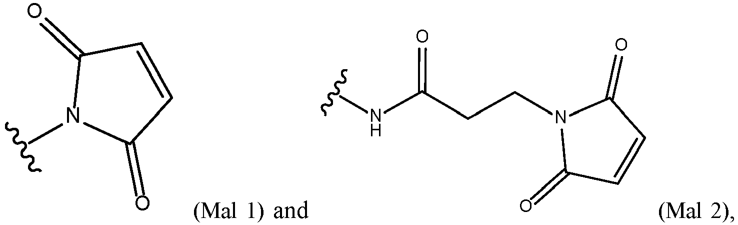 and m is comprised between 1 and 10²⁵, preferably between 1 and 2000; or
- A comprises at least one residue of poly(lactic co glycolic), B comprises at least one residue of poly(ethylene glycol), C is vinyl sulfone, and m is comprised between 1 and 10²⁵, preferably between 1 and 2000; or
- A comprises at least one residue of poly(lactic acid), B is a group of formula (II) as defined in claim 6, wherein k is 0 and p is 1, C is a maleimide of formula (Mal 1), and (Mal 2): 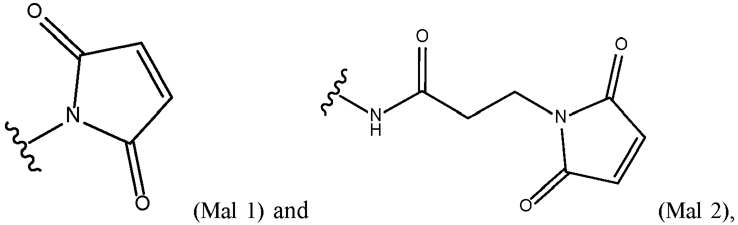 and m is comprised between 1 and 10²⁵, preferably between 1 and 2000.

8. A composition comprising a set of particles as defined in any one of claims 1 to 7, and at least one acceptable excipient, said composition being preferably a topical composition such as a topical composition in the form of a suspension, a cream, a spray, an aerosol, a butter, a stick, a gel, an ointment, a lotion, a solution, a solid, an emulsion, an oil, a lyophilizate, a milk, a powder, a paste, a wax, a mousse or a foam.

9. The composition according to claim 8, wherein said composition is a cosmetic composition, preferably a skin care, hair care, makeup, skin styling, skin color, hair styling or hair color composition.

10. A cosmetic use of a composition as defined in claim 8 or 9, for combatting and/or reducing the signs of cutaneous ageing, such as the formation of wrinkles and/or fine lines, skin sagging, loss of firmness, loss of radiance and/or evenness of the complexion, and/or for reinforcing the skin barrier.

11. A kit comprising:
- a composition as defined in claim 8 or 9,
- a washing composition, preferably a powder, a shampoo, a soap, a lotion, a solution, a solid, a scrubbing, a scraper, a mousse, a foam, a syndet, a gel, a shower gel, a spray, a mist, a wax, a strip, an enzyme composition, a detergent composition or a woven or non-woven fabric, and
- optionally an instruction guide.

## Patentansprüche

1. Partikelanordnung, die zu mindestens 20 Gew.-%, bezogen auf das Gesamtgewicht der Anordnung, aus bioadhäsiven Partikeln besteht, wobei diese bioadhäsiven Partikel durch die folgende Formel (I) dargestellt werden:
{A-[(B)ₙ-C]ₘ} (I),
worin:
- A für einen Polymerkern steht,
- B für einen linearen Linker steht,
- C für eine funktionelle Gruppe steht, ausgewählt aus einem Aldehyd, Acetal, Thiocetal, Thiol, Maleimid, Mickael-Akzeptor, Vinylsulfon, Sulfonylaziridin, Epoxid, Halogenacetyl, Isocyanat, Isothiocyanat, N-Hydroxysuccinimidester, N-Hydroxysulfosuccinimidester, Hydroxy, Amino, Ammonium, Guanidinium, Carbonsäure, Carbonsäureester, Anhydrid, Sulfonsäure, Folsäure, Biotin, Streptavidin, Avidin, Antikörpern, Einzelkettenantikörpern oder Fragmenten davon,
- n 1 ist, und
- m eine ganze Zahl von 1 bis 10²⁵, vorzugsweise von 1 bis 2000, ist und
wobei dieses bioadhäsive Partikel mindestens einen Wirkstoff X einkapselt oder daran gebunden ist, der ein UV-Filter ist.

2. Die Partikelanordnung nach Anspruch 1, wobei:
- A mindestens einen Rest eines Polymers umfasst, ausgewählt aus Poly(milchsäure), Poly(glykolsäure), Poly(milch-co-glykolsäure), Polyacrylsäure, Acrylsäure-Copolymeren, Poly(methacrylsäure), Polyalkylacrylaten (z. B. Polymethacrylaten, Polybutylacrylaten), Polyalkylcyanoacrylaten, Polyvinylacetatphthalat, Cellulose, Ethylcellulose, Celluloseacetat, Celluloseacetatphthalat, Hydroxyethylcellulose, Carboxymethylcellulose, Polyvinylalkohol, Polycaprolacton, Polycaprolacton-Crosspolymeren, Styrol-Copolymeren, Nylons, Polymethylsilsesquioxan, Poly(2-ethyl-2-oxazolin), Povidon, Alginat, Dextran, Chitosan, Poly(methylvinylether/maleinsäureanhydrid)-Copolymeren, Poly(milchsäure)-b-Poly(ethylenglykol) und Kombinationen davon.

3. Die Partikelanordnung nach Anspruch 1 oder 2, wobei B mindestens einen Rest eines Polymers umfasst, ausgewählt aus Polyalkylenglykolen wie Polyethylenglykol, Poly(methacrylsäure), Polymethacrylaten, Polyalkylcyanoacrylaten, Polyvinylacetatphthalat, Polyvinylalkohol, Povidon und Poly(methylvinylether/maleinsäureanhydrid)-Copolymeren sowie Kombinationen davon.

4. Die Partikelanordnung nach einem der Ansprüche 1 bis 3, wobei B eine Gruppe der Formel (II) ist:
-[Y-(CH₂)_{q}]ₖ-(O-CH₂-CH₂)ₚ- (II),
worin:
- Y ausgewählt ist aus -O-, -NH- und -C(O)-
- p eine ganze Zahl von 0 bis 25 ist,
- k 0 oder 1 ist,
- q eine ganze Zahl von 1 bis 10 ist, und
- p+k ungleich 0 ist.

5. Die Partikelanordnung nach einem der Ansprüche 1 bis 4, wobei C ausgewählt ist aus einem Thiol, einem Maleimid, einem Mickael-Akzeptor, einem Vinylsulfon, einem Sulfonylaziridin, einem Epoxid, einem Halogenacetyl, einem Isocyanat, einem Isothiocyanat, einem N-Hydroxysuccinimidester, einem N-Hydroxysulfosuccinimidester, einer Hydroxygruppe, einer Aminogruppe, einem Guanidinium-Ion, einem AmmoniumIon, einer Carbonsäure, einem Carbonsäureester, einem Anhydrid, einer Sulfonsäure, Folsäure, Biotin, Streptavidin, Avidin, Antikörpern, Einzelkettenantikörpern oder Fragmenten davon.

6. Die Partikelanordnung nach einem der Ansprüche 1 bis 5, wobei:
- A mindestens einen Rest eines Polymers, ausgewählt aus Poly(milchsäure) und Poly(milchsäure-co-glykolsäure), umfasst;
- B mindestens einen Rest von Polyethylenglykol umfasst oder B eine Gruppe der Formel (II) nach Anspruch 4 ist, wobei k = 0 und p = 1 ist;
- C ein Maleimid der Formel (Mal 1) und (Mal 2) ist: 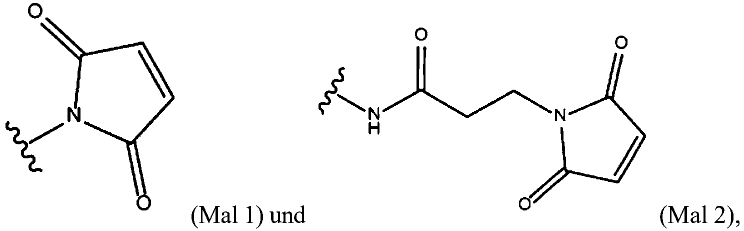 oder ein Vinylsulfon; und
- m zwischen 1 und 10²⁵, vorzugsweise zwischen 1 und 2000, liegt.

7. Die Partikelanordnung nach einem der Ansprüche 1 bis 6, wobei:
- A mindestens einen Rest aus Poly(milchsäure) umfasst, B mindestens einen Rest von Polyethylenglykol umfasst, C ein Maleimid der Formel (Mal 1) und (Mal 2) ist: 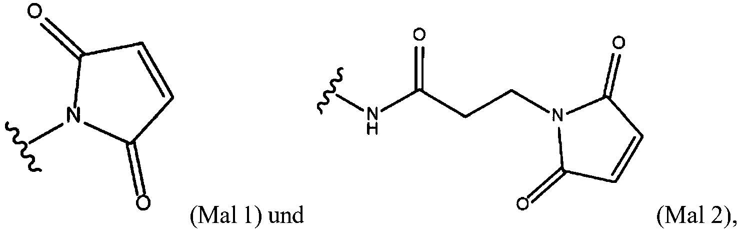 und m zwischen 1 und 10²⁵, vorzugsweise zwischen 1 und 2000, liegt; oder
- A mindestens einen Rest aus Poly(milchsäure-co-glykolsäure) umfasst, B mindestens einen Rest von Polyethylenglykol umfasst, C Vinylsulfon ist; und m zwischen 1 und 10²⁵, vorzugsweise zwischen 1 und 2000, liegt; oder
- A mindestens einen Rest aus Poly(milchsäure) umfasst, B eine Gruppe der Formel (II) nach Anspruch 6 ist, wobei k = 0 und p = 1 ist, C ein Maleimid der Formel (Mal 1) und (Mal 2) ist: 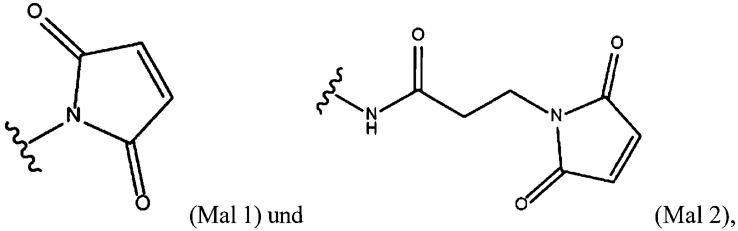 und m zwischen 1 und 10²⁵, vorzugsweise zwischen 1 und 2000, liegt.

8. Zusammensetzung, umfassend eine Partikelanordnung gemäß einem der Ansprüche 1 bis 7 und mindestens einen zulässigen Hilfsstoff, wobei es sich bei dieser Zusammensetzung vorzugsweise um eine topische Zusammensetzung handelt, beispielsweise in Form einer Suspension, Creme, eines Sprays, Aerosols, einer Butter, eines Stifts, eines Gels, einer Salbe, einer Lotion, einer Lösung, eines Feststoffs, einer Emulsion, eines Öls, eines Lyophilisats, einer Milch, eines Pulvers, einer Paste, eines Wachses, eines Mousses oder eines Schaums.

9. Die Zusammensetzung nach Anspruch 8, wobei es sich bei dieser Zusammensetzung um eine kosmetische Zusammensetzung handelt, vorzugsweise um eine Zusammensetzung für die Hautpflege, Haarpflege, Make-up, Hautstyling, Hautfärbung, Haarstyling oder Haarfärbung.

10. Kosmetische Verwendung einer Zusammensetzung nach Anspruch 8 oder 9 zur Bekämpfung und/oder Minderung der Zeichen der Hautalterung, wie z. B. der Bildung von Falten und/oder feinen Linien, Erschlaffung der Haut, Verlust der Festigkeit, Verlust der Ausstrahlung und/oder Gleichmäßigkeit des Teints, und/oder zur Stärkung der Hautbarriere.

11. Set, bestehend aus:
- einer Zusammensetzung nach Anspruch 8 oder 9,
- einer Waschzusammensetzung, vorzugsweise einem Pulver, Shampoo, Seife, Lotion, Lösung, Feststoff, Scheuermittel, Schaber, Mousse, Schaum, Syndet, Gel, Duschgel, Spray, Nebel, Wachs, Streifen, einer Enzymzusammensetzung, einer Waschmittelzusammensetzung oder einem gewebten oder nicht gewebten Stoff, und
- optional einer Gebrauchsanweisung.

## Revendications

1. Ensemble de particules comprenant au moins 20 % en poids de particules bioadhésives, par rapport au poids total de l'ensemble, lesdites particules bioadhésives sont représentées par la formule suivante (I) :
{A-[(B)ₙ-C]ₘ} (1),
dans laquelle :
- A représente un noyau polymère,
- B représente un lieur linéaire,
- C représente un groupe fonctionnel choisi parmi un aldéhyde, un acétal, thioacétal, un thiol, un maléimide, un accepteur de Mickael, une vinylsulfone, une sulfonylaziridine, un époxyde, un halogénoacétyle, un isocyanate, un isothiocyanate, un ester de N-hydroxysuccinimide, un ester de N-hydroxysulfosuccinimide, un hydroxy, un amino, un ammonium, un guanidinium, un acide carboxylique, un ester carboxylique, un anhydride, un acide sulfonique, l'acide folique, la biotine, la streptavidine, l'avidine, les anticorps, les anticorps monocaténaires ou des fragments de ceux-ci,
- n est égal à 1, et
- m est un entier allant de 1 à 10²⁵, de préférence de 1 à 2000, et
où ladite particule bioadhésive encapsule ou est liée à au moins un agent actif X, qui est un filtre UV.

2. Ensemble de particules selon la revendication 1, dans lequel :
- A comprend au moins un résidu d'un polymère choisi parmi l'acide polylactique, l'acide polyglycolique, l'acide polylactique-co-glycolique, l'acide polyacrylique, les copolymères d'acide acrylique, l'acide polyméthacrylique, les polyalkylacrylates (par exemple les polyméthacrylates, les polyacrylates de butyle), les polyalkylcyanoacrylates, le polyacétate de vinyle phtalate, la cellulose, l'éthylcellulose, l'acétate de cellulose, l'acétate de cellulose phtalate, l'hydroxyéthylcellulose, la carboxyméthylcellulose, l'alcool polyvinylique, la polycaprolactone, les crosspolymères de polycaprolactone, les copolymères de styrène, les nylons, le polyméthylsilsesquioxane, la poly-2-éthyl-2-oxazoline, la povidone, l'alginate, le dextrane, le chitosane, les copolymères de polyméthyl vinyl éther / anhydride maléique, l'acide polylactique-b-polyéthylène glycol, et des combinaisons de ceux-ci.

3. Ensemble de particules selon la revendication 1 ou 2, dans lequel B comprend au moins un résidu d'un polymère choisi parmi les polyalkylène glycols comme le polyéthylène glycol, l'acide polyméthacrylique, les polyméthacrylates, les polyalkylcyanoacrylates, le polyacétate de vinyle phtalate, l'alcool polyvinylique, la povidone, et les copolymères de polyméthyl vinyl éther / anhydride maléique, et une combinaison de ceux-ci.

4. Ensemble de particules selon l'une quelconque des revendications 1 à 3, dans lequel B est un groupe de formule (II) :
-[Y-(CH₂)_{q}]ₖ-(O-CH₂-CH₂)ₚ- (II),
dans laquelle :
- Y est choisi parmi -O-, -NH- et -C(O)-,
- p est un entier allant de 0 à 25,
- k est égal à 0 ou 1,
- q est un entier allant de 1 à 10, et
- p+k est différent de 0.

5. Ensemble de particules selon l'une quelconque des revendications 1 à 4, dans lequel C est choisi parmi un thiol, un maléimide, un maléimide, un accepteur de Mickael, une vinylsulfone, une sulfonylaziridine, un époxyde, un halogénoacétyle, un isocyanate, un isothiocyanate, un ester de N-hydroxysuccinimide, un ester de N-hydroxysulfosuccinimide, un hydroxy, un amino, un ammonium, un guanidinium, un acide carboxylique, un ester carboxylique, un anhydride, un acide sulfonique, l'acide folique, la biotine, la streptavidine, l'avidine, les anticorps, les anticorps monocaténaires ou des fragments de ceux-ci.

6. Ensemble de particules selon l'une quelconque des revendications 1 à 5, dans lequel :
- A comprend au moins un résidu d'un polymère choisi parmi l'acide polylactique et l'acide polylactique-co-glycolique ;
- B comprend au moins un résidu de polyéthylène glycol, ou B est un groupe de formule (II) tel que défini selon la revendication 4, dans laquelle k est égal à 0 et p est égal à 1 ;
- C est un maléimide de formule (Mal 1) et (Mal 2) : 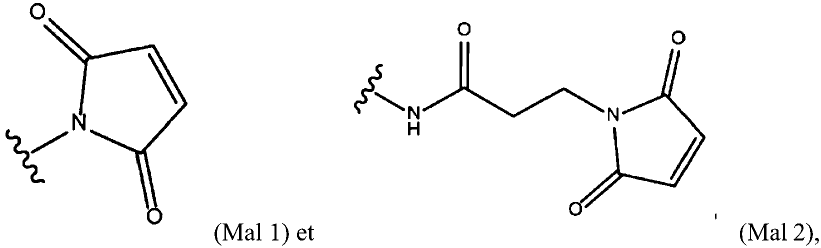 ou
une vinylsulfone ; et
- m est compris entre 1 et 10²⁵, de préférence entre 1 et 2000.

7. Ensemble de particules selon l'une quelconque des revendications 1 à 6, dans lequel :
- A comprend au moins un résidu d'acide polylactique, B comprend au moins un résidu de polyéthylène glycol, C est un maléimide de formule (Mal 1) et (Mal 2) : 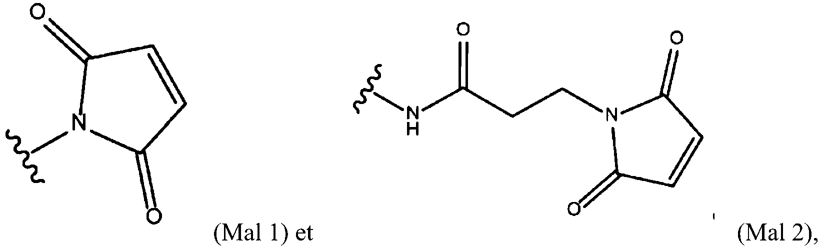 et
m est compris entre 1 et 10²⁵, de préférence entre 1 et 2000 ; ou
- A comprend au moins un résidu d'acide polylactique-co-glycolique, B comprend au moins un résidu de polyéthylène glycol, C est une vinylsulfone, et m est compris entre 1 et 10²⁵, de préférence entre 1 et 2000 ; ou
- A comprend au moins un résidu d'acide polylactique, B est un groupe de formule (II) tel que défini selon la revendication 6, dans laquelle k est égal à 0 et p est égal à 1, C est un maléimide de formule (Mal 1), et (Mal 2) : 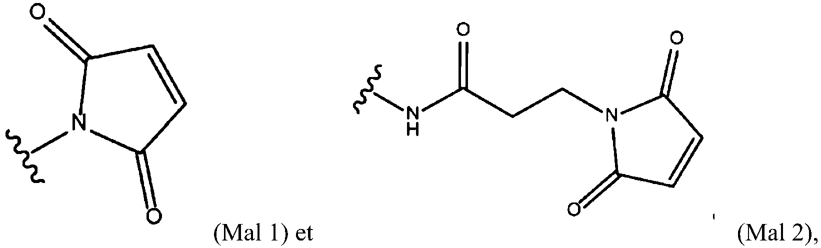 et
m est compris entre 1 et 10²⁵, de préférence entre 1 et 2000.

8. Composition comprenant un ensemble de particules tel que défini selon l'une quelconque des revendications 1 à 7, et au moins un excipient acceptable, ladite composition étant de préférence une composition topique telle qu'une composition topique sous la forme d'une suspension, d'une crème, d'un spray, d'un aérosol, d'un beurre, d'un bâtonnet, d'un gel, d'une pommade, d'une lotion, d'une solution, d'un pain, d'une émulsion, d'une huile, d'un lyophilisat, d'un lait, d'une poudre, d'une pâte, d'une cire, d'une mousse ou d'une écume.

9. Composition selon la revendication 8, où ladite composition est une composition cosmétique, de préférence une composition de soin cutané, de soin capillaire, de maquillage, de styling cutané, de teinture cutanée, de styling capillaire ou de teinture capillaire.

10. Utilisation cosmétique d'une composition telle que définie selon la revendication 8 ou 9, pour combattre et/ou réduire les signes de vieillissement cutané, tels que la formation de rides et/ou de ridules, le relâchement cutané, la perte de fermeté, la perte d'éclat et/ou l'absence d'uniformité du teint, et/ou le renforcement de la barrière cutanée.

11. Kit comprenant :
- une composition telle que définie selon la revendication 8 ou 9,
- une composition de lavage, de préférence une poudre, un shampooing, un savon, une lotion, une solution, un pain, un gommage, un exfoliant, une mousse, une écume, un détergent synthétique, un gel, un gel douche, un spray, une brume, une cire, une bande, une composition enzymatique, une composition de détergence, ou une lingette tissée ou non tissée, et
- éventuellement une notice d'utilisation.
